(19) Europäisches Patentamt
European Patent Office
Office européen des brevets

(11) Publication number: **0 307 141 B1**

# (12) EUROPEAN PATENT SPECIFICATION

(45) Date of publication of patent specification: **13.01.93**

(51) Int. Cl.⁵: **A61K 31/42**, A61K 31/425, A61K 31/395, A61K 31/40, A61K 31/44, A61K 31/445, A61K 31/435, A61K 31/55, C07D 413/04, C07D 417/04, C07D 451/02

(21) Application number: **88308126.7**

(22) Date of filing: **01.09.88**

## (54) Oxazoles and thiazoles for the treatment of senile dementia.

(30) Priority: **10.09.87 GB 8721343**
**27.01.88 GB 8801759**

(43) Date of publication of application:
**15.03.89 Bulletin 89/11**

(45) Publication of the grant of the patent:
**13.01.93 Bulletin 93/02**

(84) Designated Contracting States:
**AT BE CH DE ES FR GB IT LI LU NL SE**

(56) References cited:
**EP-A- 0 234 011**
**EP-A- 0 244 018**
**EP-A- 0 296 721**
**DE-A- 3 519 261**
**EP-A- 0 239 309**
**EP-A- 0 261 763**
**BE-A- 904 945**

(73) Proprietor: **MERCK SHARP & DOHME LTD.**
**Hertford Road**
**Hoddesdon Hertfordshire EN11 9BU(GB)**

(72) Inventor: **Baker, Raymond**
**Bulls Cottage Green Tye**
**Much Hadham Hertfordshire(GB)**
Inventor: **Snow, Roger J.**
**43 Lancing Road Newbury Park**
**Ilford Essex(GB)**
Inventor: **Saunders, John**
**13 The Ridings Thorley Park**
**Bishops Stortford Hertsfordshire(GB)**
Inventor: **Showell, Graham A.**
**5 Beehive Lane**
**Welwyn Garden City Hertfordshire(GB)**

(74) Representative: **Hesketh, Alan, Dr.**
**European Patent Department Merck & Co., Inc. Terlings Park Eastwick Road**
**Harlow Essex, CM20 2OR(GB)**

EP 0 307 141 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a class of substituted oxazole and thiazole compounds which stimulate central muscarinic acetylcholine receptors and therefore are useful in the treatment of neurological and mental illnesses whose clinical manifestations are due to cholinergic deficiency. Such diseases include presenile and senile dementia (also known as Alzheimer's disease and senile dementia of the Alzheimer type respectively), Huntington's chorea, tardive dyskinesia, hyperkinesia, mania and Tourette Syndrome. Alzheimer's disease, the most common dementing illness, is a slowly progressive neurological disorder characterised by marked deficits in cognitive functions including memory, attention, language and visual perception capabilities. The compounds of this invention are also useful analgesic agents and therefore useful in the treatment of severe painful conditions such as rheumatism, arthritis and terminal illness.

Compounds capable of enhancing muscarinic cholinergic transmission in the cortex should be beneficial in reversing the cholinergic deficiency in Alzheimer's disease and other diseases related to cholinergic dysfunction. However, most muscarinic ligands, including acetylcholine itself, are quaternary ammonium compounds incapable of penetrating the blood-brain barrier to any clinically significant extent following peripheral (e.g. oral) administration. Such agents fail to stimulate the desired central sites but instead induce undesired side-effects mediated exclusively by peripherally-located muscarinic acetylcholine receptors.

The oxazole and thiazole compounds of the present invention stimulate cholinergic transmission but, being either secondary or tertiary amines with physiochemical properties (lipophilicity and pKa) consistent with CNS penetrability, can stimulate those central sites implicated in neurodegenerative disorders. It is believed that the enhancement of cholinergic transmission demonstrated by the compounds of this invention is achieved either directly by stimulating postsynaptic receptors, or indirectly by potentiating acetylcholine release.

Most of the oxazoles and thiazoles which possess this activity are novel compounds, although certain piperidinyl- and tetrahydropyridinyl-substituted oxazoles and thiazoles are known from U.S. Patent 4,650,805 and from EP-A-0244018. These compounds are stated to be of use in the treatment of psychosis and disorders of the central nervous system, such as schizophrenia, Parkinson's disease and depression. That activity does not, however, suggest that any of these compounds could be muscarinic agonists.

In addition, EP-A-0261763, which was published on 30 March 1988, describes a class of compounds which include oxazoles and thiazoles having particular exo-azabicyclic substituents. These compounds are stated to be of potential use in the treatment and/or prophylaxis of dementia in mammals.

Furthermore, EP-A-0296721, which was published on 28 December 1988, describes a class of piperidinyl- and tetrahydropyridinyl- substituted heterocycles which include oxazoles and thiazoles having particular substituents on the heterocycle. These compounds are stated to have acetylcholine agonist activity.

The present invention provides the use of a 1,3-oxazole or 1,3-thiazole of structural formula I, or a pharmaceutically acceptable salt or prodrug thereof:

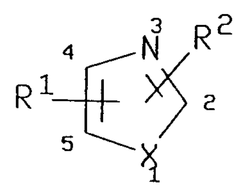

(I)

(I)

wherein

X represents oxygen or sulphur;

$R^1$ represents a non-aromatic azacyclic or azabicyclic ring system which system may be fused, spiro or bridged, containing one nitrogen atom as the sole heteroatom and from 4 to 10 ring atoms; and

$R^2$ represents hydrogen, halogen, $-CF_3$, $-OR^7$, $-NR^7R^8$, $-NHOR^7$, $-NHNH_2$, -CN, $-CO_2R^7$ or $-CONR^7R^8$, or a substituted or unsubstituted, saturated or unsaturated hydrocarbon group selected from $C_{1-15}$ alkyl, $C_{2-15}$ alkenyl, $C_{2-15}$ alkynyl, aryl and aralkyl; wherein $R^7$ and $R^8$ independently represent hydrogen or $C_{1-2}$ alkyl;

provided that:

(a) when $R^2$ is hydrogen and $R^1$ is in the 2-position, then $R^1$ is in the endo configuration; and

(b) when $R^2$ is methyl and $R^1$ is in the 2-position, then $R^1$ does not represent an unsubstituted exo-1-azabicyclo[2.2.1]hept-3-yl, exo-1-azabicyclo[3.2.1]oct-3-yl or exo-1-azabicyclo[3.2.1]oct-6-yl group;

(c) when $R^1$ is 1,2,5,6-tetrahydropyrid-3-yl or piperid-3-yl, optionally substituted on a ring carbon or nitrogen atom thereof by $C_{1-4}$ alkyl, then $R^2$ is other than hydrogen, $-CF_3$, $-OR^7$ or $C_{1-6}$ alkyl optionally substituted by halogen, -OH or phenyl which may itself be optionally substituted by halo, $C_{1-6}$ alkoxy, $C_{1-6}$ alkyl or $-CF_3$;

for the preparation of a medicament useful for the treatment of neurological and mental illnesses whose clinical manifestations are due to involvement of cholinergic neurones.

The present invention also provides a novel 1,3-oxazole or 1,3-thiazole represented by the structural formula II, or a salt or prodrug thereof:

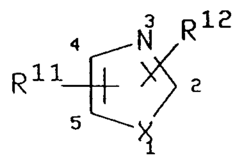

(II)

(II)

wherein

X is oxygen or sulphur;

$R^{11}$ represents a non-aromatic azacyclic or azabicyclic ring system which system may be fused, spiro or bridged, containing one nitrogen atom as the sole heteroatom and from 4 to 10 ring atoms ; and

$R^{12}$ represents hydrogen, halogen, $-CF_3$, $-OR^7$, $-NR^7R^8$, $-NHOR^7$, $-NHNH_2$, -CN, $-CO_2R^7$ or $-CONR^7R^8$ or an optionally substituted, saturated or unsaturated hydrocarbon group selected from $C_{1-15}$ alkyl, $C_{2-15}$ alkenyl, $C_{2-15}$ alkynyl, aryl and aralkyl; wherein $R^7$ and $R^8$ are as defined above;

provided that when $R^{12}$ is present in the 2-position and represents amino, alkylamino, dialkylamino or alkylcarbonylamino in which the alkyl moieties have from 1 to 4 carbon atoms, then $R^{11}$, when present in the 4-position, does not represent optionally N-substituted piperidin-3-yl or 1,2,5,6-tetrahydropyridin-3-yl;

provided also that:

(a) when $R^{12}$ is hydrogen and $R^{11}$ is in the 2-position, then $R^{11}$ is in the endo configuration; and

(b) when $R^{12}$ is methyl and $R^{11}$ is in the 2-position, then $R^{11}$ does not represent an unsubstituted exo-1-azabicyclo[2.2.1]hept-3-yl, exo-1-azabicyclo[3.2.1]oct-3-yl or exo-1-azabicyclo[3.2.1]oct-6-yl group; and

(c) when $R^1$ is 1,2,5,6-tetrahydropyrid-3-yl or piperid-3-yl, optionally substituted on a ring carbon or nitrogen atom thereof by $C_{1-4}$ alkyl, then $R^2$ is other than hydrogen, $-CF_3$, $-OR^7$ or $C_{1-6}$ alkyl optionally substituted by halogen, -OH or phenyl which may itself be optionally substituted by halo, $C_{1-6}$ alkoxy, $C_{1-6}$ alkyl or $-CF_3$.

The azacyclic or azabicyclic ring system for the groups $R^1$/$R^{11}$ is a non-aromatic ring system containing one nitrogen atom as the sole heteroatom and from 4 to 10 ring atoms, preferably from 5 to 8 ring atoms. Preferably, the ring system contains a tertiary amino nitrogen atom in a caged structure. The bicyclic systems may be fused, spiro or bridged. Preferably, the nitrogen atom is at a bridgehead in a bicyclic system. Examples of suitable azacyclic or azabicyclic ring systems include the following:

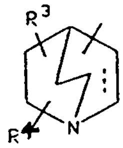 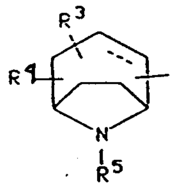 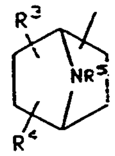

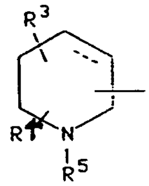 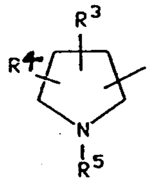 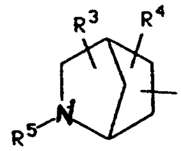

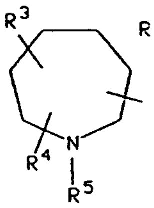 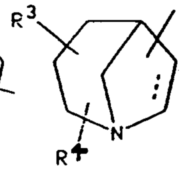 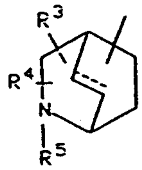 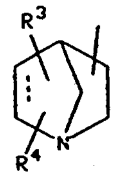 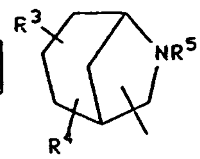

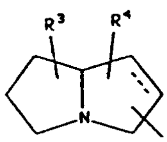 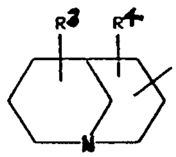 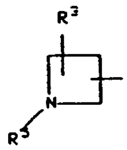 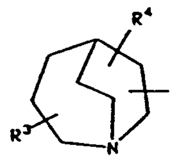 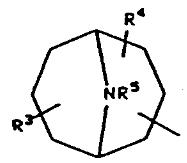

wherein the broken line represents an optional chemical bond;

the substituents $R^3$ and $R^4$ may be present at any position, including the point of attachment to the oxazole or thiazole ring, and independently represent hydrogen, $C_{1-4}$ alkyl, halo, $C_{1-4}$ alkoxy, hydroxy or carboxy; or $R^3$ and $R^4$ together represent carbonyl; and

the group $R^5$ represents hydrogen or $C_{1-4}$ alkyl.

It will be appreciated that the nitrogen atom in the azacyclic or azabicyclic ring system will carry a lone pair of electrons.

Suitably the group $R^3$ is hydrogen or methyl; and $R^4$ is hydrogen, methyl or hydroxy. Preferably one or both of $R^3$ and $R^4$ is hydrogen.

Preferably the group $R^5$ represents hydrogen or methyl.

Suitably the azacyclic or azabicyclic ring system is a pyrrolidine, piperidine, tetrahydropyridine, azanorbornane, quinuclidine or 1-azabicyclo[3.2.1]octane ring system. Preferred values for the azacyclic or azabicyclic ring system are tetrahydropyridine, 1-azanorbornane and quinuclidine, in particular either unsubstituted or substituted with methyl or hydroxy.

The substituent $R^2$ or $R^{12}$ on the oxazole or thiazole ring may be a substituent of low lipophilicity. The term "low lipophilicity" is intended to indicate that the group has a Rekker f value (hydrophobic fragment constant; see R. F. Rekker, "The Hydrophobic Fragmental Constant", Elsevier, 1977) of not greater than 1.5. For eyample, the methyl group has a value of 0.7 and the ethyl group a value of 1.26.

Thus the substituent of low lipophilicity may be hydrogen, halogen, $-CF_3$, $-OR^7$, $-NR^7R^8$, $-NHOR^7$, $-NHNH_2$, -CN, $-CO_2R^7$, $-CONR^7R^8$, $C_{2-5}$ alkenyl, $C_{2-5}$ alkynyl, $C_{1-2}$ alkyl, or $C_{1-2}$ alkyl substituted with $-OR^7$, $-NR^7R^8$, $-SR^7$, $-CO_2R^7$, $-CONR^7R^8$ or halogen; wherein $R^7$ and $R^8$ are as defined above with respect to formula I.

Alternatively the group $R^2$ or $R^{12}$ may represent an optionally substituted saturated hydrocarbon group having at least three carbon atoms, or unsaturated hydrocarbon group having at least 6 carbon atoms selected from $C_{1-15}$ alkyl, $C_{2-15}$ alkenyl, $C_{2-15}$ alkynyl, aryl or aralkyl. The alkyl, alkenyl or alkynyl groups may be straight, branched or cyclic groups. Suitably the alkyl group comprises from 1 to 6 carbon atoms. The hydrocarbon group may carry one or more substituents. Suitable substituents for the hydrocarbon group include halogen, $-OR^6$, $-CF_3$, $-NR^6R^9$, $-NO_2$, optionally substituted aryl, keto, $-SR^6$, $-SOR^6$, $-SO_2R^6$, $-CO_2R^6$ and $-CONR^6R^9$; wherein $R^6$ is hydrogen or $C_{1-6}$ alkyl and $R^9$ is hydrogen, $C_{1-6}$ alkyl or $-COCH_3$.

Substituents most suitable for the aryl group include chloro, bromo, methoxy, $C_{1-6}$ alkyl, methoxycarbonyl, trifluoromethyl, nitro and $-NR^6R^7$.

Preferably the group $R^2$ or $R^{12}$ is hydrogen, halogen, $-CF_3$, $-OR^7$, $-NR^7R^8$, $-NHNH_2$, -CN, $-CO_2R^7$,

$-CONR^7R^8$, phenyl($C_{1-3}$)alkyl, $C_{3-6}$ cycloalkyl, adamantyl, $C_{2-6}$ alkenyl, $C_{2-6}$ alkynyl, $C_{1-6}$ alkyl, or $C_{1-6}$ alkyl substituted with $-OR^6$, $-NHR^6$, $-SR^6$, $-CO_2R^6$, $-CON(R^6)_2$ or halogen. Particular values of this group are hydrogen, methyl, ethyl, isopropyl, cyclopropyl, benzyl, 1-phenylethyl, adamantyl, amino, methoxycarbonyl and ethoxycarbonyl. Preferred values are hydrogen, methyl, ethyl and amino.

One group of prodrugs of compounds of this invention have a substituent on the oxazole or thiazole ring which is hydrolysable in vivo to an amino group.

Groups which are hydrolysable in vivo to an amino group on the compounds of this invention may be readily ascertained by administering the compound to a human or animal and detecting, by conventional analytical techniques, the presence of the corresponding compound having an amino substituent in the urine of a human or animal. Examples of such groups include, for example, amido and urethane substituents, in particular a group of formula -NH.Q, wherein Q represents CHO, COR or $CO_2R$, and R represents an optionally substituted hydrocarbon group.

In this context, the hydrocarbon group R includes groups having up to 20 carbon atoms, suitably up to 10 carbon atoms, conveniently up to 6 carbon atoms. Suitable groups R include $C_{1-9}$ alkyl, $C_{2-6}$ alkenyl, $C_{2-6}$ alkynyl, $C_{3-7}$ cycloalkyl, $C_{3-7}$ cycloalkyl($C_{1-6}$)alkyl, aryl, and aryl($C_{1-6}$)alkyl. The alkyl group R may be straight or branched chain and may contain, for example, up to 12 carbon atoms, suitably from 1 to 6 carbon atoms. In particular the group may be substituted methyl, ethyl, n- or iso-propyl, n-, sec-, iso- or tert-butyl, n- or iso-heptyl, or n- or iso-octyl. Suitable cycloalkyl groups include cyclopentyl and cyclohexyl. The aryl group R includes phenyl and naphthyl optionally substituted with up to five, preferably up to three, substituent groups.

One sub-class of novel compounds within the scope of the present invention is represented by formula III:

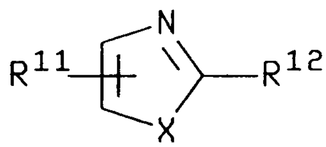

(III)

wherein X, $R^{11}$ and $R^{12}$ are as defined above; in particular wherein $R^{11}$ represents tetrahydropyridine, 1-azanorbornane, quinuclidine or 1-azabicyclo[3.2.1]octane, any of which groups $R^{11}$ may be optionally substituted with $C_{1-3}$ alkyl, or hydroxy; and $R^{12}$ represents hydrogen, $C_{1-6}$ alkyl (preferably methyl), phenyl($C_{1-3}$)alkyl, $C_{3-6}$ cycloalkyl, amino, hydroxy, or $C_{1-3}$ alkoxy. Preferably $R^{11}$ represents tetrahydropyridine, quinuclidine or 1-azanorbornane.

A further subclass of compounds of this invention is represented by the formula IV:

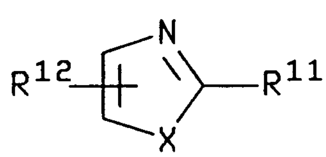

(IV)

wherein X, $R^{11}$ and $R^{12}$ are as defined above.

Within formula IV preferably $R^{11}$ represents tetrahydropyridine, quinuclidine, 1-azanorbornane or 1-azabicyclo[3.2.1]octane, any of which groups $R^{11}$ may be optionally substituted with $C_{1-3}$ alkyl or hydroxy. Preferably $R^{12}$ is hydrogen, amino, hydroxy, methyl or ethyl.

Specific compounds within the scope of the present invention include:

3-[4-(2-amino-1,3-thiazol)-yl]quinuclidine;

3-[4-(1,3-thiazol)-yl]quinuclidine;

3-[4-(2-amino-1,3-thiazol)-yl]pyrrolidine;

3-[4-(2-amino-1,3-thiazol)-yl]-1-methylpyrrolidine;
3-[4-(2-methyl-1,3-thiazol)-yl]quinuclidine;
3-[4-(2-hydroxy-1,3-thiazol)-yl]quinuclidine;
3-[5-(2-methyl-1,3-thiazol)-yl]quinuclidine;
3-[4-(2-amino-1,3-thiazol)-yl]-1-azabicyclo[2.2.1]heptane;
3-[5-(2-methyl-1,3-oxazol)-yl]quinuclidine;
3-[5-(2-methyl-1,3-oxazol)-yl]-1-azabicyclo[2.2.1]heptane;
3-[4-(2-methyl-1,3-oxazol)-yl]quinuclidine;
3-[4-(2-amino-1,3-oxazol)-yl]quinuclidine;
3-[2-(4-methyl-1,3-thiazol)-yl]quinuclidine;
3-[2-(4-methyl-1,3-oxazol)-yl]quinuclidine;
3-[2-(5-methyl-1,3-oxazol)-yl]quinuclidine;
3-[2-(4-methyl-1,3-thiazol)-yl]-1-azabicyclo[2.2.2]oct-2-ene;
3-hydroxy-3-[2-(4-methyl-1,3-oxazol)-yl]quinuclidine;
(+)-3-[2-(4-methyl-1,3-oxazol)-yl]quinuclidine;
(-)-3-[2-(4-methyl-1,3-oxazol)-yl]quinuclidine;
3-hydroxy-3-[2-(1,3-oxazol)-yl]quinuclidine;
3-hydroxy-3-[2-(4-ethyl-1,3-oxazol)-yl]quinuclidine;
3-[2-(4-ethyl-1,3-oxazol)-yl]quinuclidine;
3-hydroxy-3-[2-(4-methyl-1,3-thiazol)-yl]quinuclidine;
1-methyl-3-[2-(5-methyl-1,3-oxazol)-yl]piperidine;
1-methyl-3-[2-(5-methyl-1,3-oxazol)-yl]-1,2,5,6-tetrahydropyridine;
3-[2-(5-methyl-1,3-oxazol)-yl]-1,2,5,6-tetrahydropyridine;
1-methyl-3-[4-(2-methyl-1,3-thiazol)-yl]-1,2,5,6-tetrahydropyridine;
3-[4-(2-methyl-1,3-thiazol)-yl]-1,2,5,6-tetrahydropyridine;
1-methyl-3-[4-(2-methyl-1,3-oxazol)-yl]-1,2,5,6-tetrahydropyridine;
3-[4-(2-methyl-1,3-oxazol)-yl]-1,2,5,6-tetrahydropyridine;
1-methyl-3-[2-(4-methyl-1,3-oxazol)-yl]-1,2,5,6-tetrahydropyridine;
1-methyl-3-[5-(2-methyl-1,3-oxazol)-yl]-1,2,5,6-tetrahydropyridine;
3-[2-(4-methyl-1,3-oxazol)-yl]-1,2,5,6-tetrahydropyridine;
3-[5-(2-methyl-1,3-oxazol)-yl]-1,2,5,6-tetrahydropyridine;
and salts and prodrugs thereof.

Most of the compounds of this invention have at least one asymmetric centre and often more than one; and can therefore exist as both enantiomers and diastereoisomers. In particular, those compounds possessing an unsymmetrical azabicyclic ring system may exist as exo and endo diastereoisomers. The oxazoles and thiazoles having a hydroxy substituent are likely to exist in a tautomeric form having a keto group, for example:

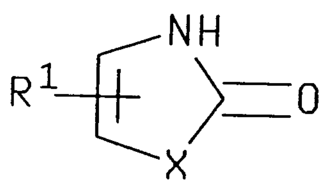

It is to be understood that the invention covers all such isomers, mixtures thereof, and tautomeric forms.

Also included within the scope of the present invention are salts of the novel compounds. It will be appreciated that salts of the compounds for use in medicine will be non-toxic pharmaceutically acceptable salts. Other salts may, however, be useful in the preparation of the compounds of the invention or their non-toxic pharmaceutically acceptable salts. Acid addition salts, for example, may be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid or phosphoric acid. Where the novel compound carries a carboxylic acid group the invention also contemplates salts thereof, preferably non-toxic pharmaceutically acceptable salts thereof, such as the sodium, potassium and calcium salts thereof.

Salts of amine groups may also comprise the quaternary ammonium salts in which the amino nitrogen atom carries an alkyl, alkenyl, alkynyl or aralkyl group. Such quaternary ammonium derivatives penetrate poorly into the central nervous system and are therefore useful as peripherally selective muscarinic agents,

useful for example as antispasmodic agents, agents to reduce gastric acid secretion, agents to block the muscarinic actions of acetylcholinesterase inhibitors in the treatment of myasthenia gravis and as agents to co-administer with muscarinic agonists in Alzheimer's disease.

It is believed that those compounds of formula I above which directly stimulate post-synaptic receptors are particularly useful as analgesic agents.

This invention therefore also provides a pharmaceutical composition comprising a compound of formula II above and a pharmaceutically acceptable carrier.

It may, where appropriate, be advantageous, in order to reduce unwanted peripherally mediated side-effects, to incorporate into the composition a peripherally acting cholinergic antagonist (or anti-muscarinic agent). Thus the compounds of formula I may advantageously be administered together with a peripheral cholinergic antagonist such as N-methylscopolamine, N-methylatropine, propantheline, methantheline or glycopyrrolate.

The compounds of formula I can be administered orally, parenterally or rectally at a daily dose of about 0.01 to 10 mg/kg of body weight, preferably about 0.1 to 1 mg/kg, and may be administered on a regimen of 1-4 times a day. When a cholinergic antagonist is administered, it is incorporated at its conventional dose.

The pharmaceutical formulations of this invention preferably are in unit dosage forms such as tablets, pills, capsules, powders, granules, sterile parenteral solutions or suspensions, or suppositories for oral, parenteral or rectal administration. For preparing solid compositions such as tablets, the principal active ingredient is mixed with a pharmaceutical carrier, e.g. conventional tabletting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups and flavoured emulsions with edible oils such as cottonseed oil, sesame oil, coconut oil and peanut oil, as well as elixirs and similar pharmaceutical vehicles. Suitable dispersing or suspending agents for aqueous suspension include synthetic and natural gums such as tragacanth, acacia, alginate, dextran, sodium carboxymethylcellulose, methylcellulose, polyvinyl-pyrrolidone and gelatin.

The novel compounds of this invention may be prepared by a process which comprises reacting a compound of formula $R^aCO.CH_2Y$, wherein Y represents bromo, chloro, or iodo; with a compound of formula $R^bCX.NH_2$, wherein X is as hereinbefore defined, and one of $R^a$ and $R^b$ represents a group $R^{11}$ and the other represents a group $R^{12}$. The reaction may be carried out in a suitable solvent, such as ethanol or acetic acid, or in a melt at a temperature of from 40°C to 140°C. This process is better suited to the preparation of thiazoles but may also be employed for oxazoles (see Sharanin, Zh. Org. Chim., 1980, 16, 2185; and Kerr et al., J.Am.Chem.Soc.,1960, 82, 186). The α-halocarbonyl compounds of formula $R^aCO.CH_2Y$ may be prepared by halogenation of a methyl ketone $R^aCOCH_3$.

The novel compounds of the invention may also be prepared by cyclisation of an acylaminoketone of formula $R^aCOCH_2NHCOR^b$. If the cyclisation is carried out with the insertion of sulphur, in the presence of a dehydrating sulphide such as phosphorus pentasulphide, the product of the reaction is a thiazole. In this case the reaction is carried out either in a melt or in a suitable solvent, such as toluene, at a temperature of from 40°C to 140°C. Alternatively if the compound $R^aCOCH_2NHCOR^b$ is cyclised in the presence of a dehydrating agent such as concentrated sulphuric acid, polyphosphoric acid, anhydrous hydrogen fluoride, phosphorus pentachloride, phosphorus oxychloride or thionyl chloride, the product of the reaction is an oxazole. In this case, the reaction may be carried out either in the absence of solvent or in a suitable solvent such as acetic anhydride. The acylaminoketone of formula $R^aCOCH_2NHCOR^b$ may be prepared by

acylation of an aminoketone $R^aCOCH_2NH_2$ which in turn may be prepared from an $\alpha$-halocarbonyl compound $R^aCOCH_2Y$ described above, by reaction with ammonia or sodium azide followed by reduction. The aminoketone $R^aCOCH_2NH_2$ may also be prepared by means of a Neber rearrangement reaction, which involves treatment of the corresponding oxime tosylate $R^aC(=N\text{-}OTs)CH_3$ with a strong base such as potassium ethoxide. Alternatively, the aminoketone $R^aCOCH_2NH_2$ may be prepared by reacting an acid chloride of formula $R^aCOCl$ with an anion of an alkyl isocyanate followed by acid hydrolysis.

A further method for the preparation of the novel oxazoles and thiazoles of this invention comprises the dehydroxylation of a compound of formula V:

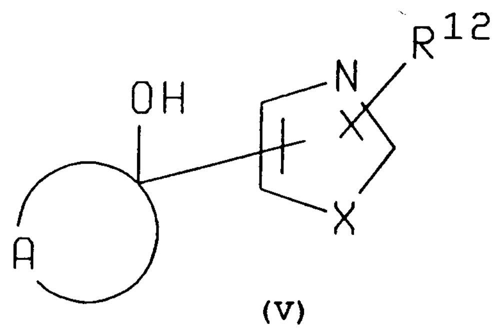

(V)

wherein $R^{12}$ and X are as defined above, and A represents the residue of an azacyclic or azabicyclic ring. The hydroxy group in formula V may be removed by chlorination and elimination, followed by hydrogenation. For example, chlorination and elimination may be effected by treatment with phosphorus oxychloride in the presence of triethylamine, or with thionyl chloride followed by DBN. The unsaturated product may then be hydrogenated under conventional conditions, such as over 10% palladium/carbon in methanol. Alternatively, the compound of formula V may be dehydroxylated by the use of thionyl chloride followed by treatment with tributyltin hydride in a solvent such as tetrahydrofuran in the presence of a radical initiator such as azabisisobutyronitrile.

The compound of formula V may be prepared by reaction of a ketone compound of formula VI with a metal derivative of an oxazole or thiazole of formula VII:

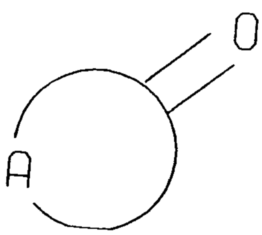

(VI)

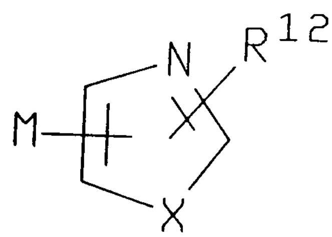

(VII)

wherein A, $R^{12}$ and X are as defined above, and M represents a metal atom, for example lithium. The lithium derivative for instance may be prepared by reacting the corresponding chloro-substituted oxazole or thiazole with n-butyl lithium.

For the preparation of a compound of formula VII wherein the lithium atom represented by the group M is present in the 2-position of the oxazole or thiazole nucleus, it may be appropriate to react the corresponding oxazole or thiazole unsubstituted in the 2-position directly with n-butyl lithium. The preparation of the required 2-unsubstituted oxazole or thiazole starting material is illustrated in general terms by a method for preparing 2-unsubstituted oxazoles VIII described by J.W. Cornforth and R.H. Cornforth in J. Chem. Soc., 1953, 93. This method essentially involves reacting the $\alpha$-chloro-$\beta$-ketoester IX with ammonium formate in the presence of formic acid, followed by decarboxylation of the resulting ester X under basic conditions with subsequent heating to reflux temperature in quinoline in the presence of a copper catalyst:

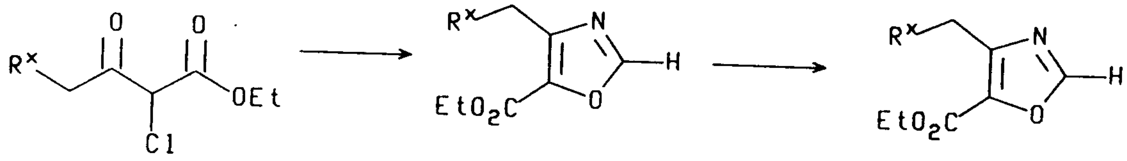

(IX) (X) (VIII)

wherein $R^x$ is a group such that $-CH_2R^x$ represents the group $R^{12}$ as defined above.

The novel oxazoles of this invention may be prepared by cyclisation of a ketal of formula $R^aCOCH_2NHCR^bR^fR^g$ in which $R^f$ and $R^g$ independently represent $C_{1-4}$ alkoxy. The reaction is conveniently carried out by heating the ketal in the presence of a suitable mineral acid catalyst at a temperature in the region of 140 °C. The ketal may in turn be prepared by reacting the aminoketone of formula $R^aCOCH_2NH_2$ with an orthoester of formula $R^bCR^fR^gR^h$ in which $R^f$, $R^g$ and $R^h$ independently represent $C_{1-4}$ alkoxy. Under appropriate reaction conditions, the cyclisation will take place spontaneously in situ, without the necessity for isolation of the intermediate ketal of formula $R^aCOCH_2NHCR^bR^fR^g$.

The novel oxazoles of the present invention may also be prepared by reacting an aldehyde of formula $R^aCHO$ with the anion of an isonitrile of formula $L\text{-}CH_2N=C(Z)R^b$, where L is a leaving group such as toluenesulphonyl, and Z is methoxy or methylthio. Suitable solvents include tetrahydrofuran, dimethylsulphoxide and dimethoxyethane.

The novel oxazoles of this invention in which the group $R^{12}$ represents an optionally monosubstituted methyl group in the 5-position may be prepared by the cyclisation of a propargyl amide of formula XI:

$R^{11}CONHCH_2C{\equiv}CR^x$ (XI)

wherein $R^{11}$ and $R^x$ are as defined above. The cyclisation may be carried out for example by treatment of the compound XI with a catalyst such as mercuric acetate in acetic acid.

The novel oxazoles of this invention in which the group $R^{12}$ represents an optionally monosubstituted methyl group in the 4-position may be prepared by a process which comprises reacting a cyano compound of formula $R^{11}CN$ with a propargyl alcohol of formula $HOCH_2C{\equiv}CR^x$, wherein $R^{11}$ and $R^x$ are as defined above. The reaction is conveniently carried out in the presence of an acidic catalyst such as, for example, sulphuric acid.

After any of the above described processes is complete, one substituent of low lipophilicity can be converted to another. For example an amino group may be converted to chloro, or hydrazo, $-NHNH_2$, via the intermediacy of diazonium, $-N^2$. Similarly, a chloro substituent may be converted, by reaction with a nucleophile such as methoxide, to methoxy, which can be converted to hydroxy under acidic conditions; and alkoxycarbonyl groups may be converted, via carboxy, to an amino substituent, $-NH_2$.

In any of the above reactions it may be necessary and/or desirable to protect any sensitive groups in the compounds. For example, if $R^a$ and/or $R^b$ include amino, carboxy, hydroxy or thiol groups, these may be protected in conventional manner. Thus, suitable protecting groups for hydroxy groups include silyl groups such as trimethylsilyl or tert-butyldimethylsilyl, and etherifying groups such as tetrahydropyranyl; and for amino groups include benzyloxycarbonyl and tert-butyloxycarbonyl. Carboxy groups are preferably protected in a reduced form such as in the form of their corresponding protected alcohols, which may be subsequently oxidised to give the desired carboxy group. Thiol groups may be protected by disulphide formation, either with the thiol itself or with another thiol to form a mixed disulphide. The protecting groups may be removed at any convenient stage in the synthesis of the desired compound according to conventional techniques.

The following Examples illustrate the preparation of compounds according to the invention. Each of the compounds of the Examples demonstrates an affinity for the muscarinic receptor, having an $IC_{50}$ - (concentration required to displace 50% of specific [$^3$H]-N-methylscopolamine binding from rat cortical membrane preparations) significantly lower than 100$\mu$M. Penetrability into the central nervous system of compounds of this invention was assessed by a measurable displacement of radioligand binding using standard "ex-vivo" binding techniques (Ref: J. Neurosurg., 1985, 63, 589-592).

In the Examples, all temperatures are in °C; THF is tetrahydrofuran; and ether is diethyl ether.

EXAMPLE 1

3-[4-(2-Amino-1,3-thiazol)-yl]quinuclidine dihydrochloride.

(a) 3-Acetyl quinuclidine

Lithium hydroxide monohydrate (0.97 g, 0.023 mol) and 3-methoxycarbonylquinuclidine [4.0 g, 0.023 mol, prepared by the method of C.A. Grob and E. Renk, Helv. Chim. Acta (1954), 196, 1689] were heated under reflux in methanol (30 ml) for 24 hours. The solvent was evaporated and the residue dried in vacuo over phosphorus pentoxide to give 3-carboxyquinuclidine lithium salt (3.70 g) as a colourless solid.

A solution of methyl lithium (16.6 ml of a 1.4 M solution in diethyl ether, 0.023 mol) was added dropwise to a cooled (0°C), stirred suspension of 3-carboxyquinuclidine lithium salt (3.70 g, 0.023 mol) in dry tetrahydrofuran (50 ml) under an atmosphere of nitrogen.

After 18 hours at room temperature, water (50 ml) was added followed by diethyl ether (50 ml). The mixture was stirred vigorously for 15 minutes, the organic layer was separated and the aqueous re-extracted with diethyl ether (2 x 50 ml). The combined organics were dried (sodium sulphate), evaporated to dryness in vacuo to give 3-acetyl-quinuclidine as a colourless oil (1.21 g, 34%); $\nu$ max (liquid film) $1700cm^{-1}$ (C=O); m/e 153 ($M^+$); $\delta$ (360 MHz, $CDCl_3$) 1.32 - 1.46 and 1.63 - 1.69 (each 2H, each m, 5 and $8CH_2$); 2.16(3H, s, $COCH_3$); 2.16 - 2.19 (1H, m, 4CH); 2.60 - 2.65 (1H, m, 3CH); 2.68 - 2.90 (5H, m, 2CH, $6CH_2$ and $7CH_2$); 3.32 (1H, ddd, J = 2,6,14 Hz, 2CH).

(b) 3-[4-(2-Amino-1,3-thiazol)-yl] quinuclidine dihydrochloride

A mixture of 3-acetylquinuclidine (0.45 g, 2.9 mmol), iodine (0.736 g, 2.9 mmol) and thiourea (0.447 g, 5.9 mmol) was heated on a steam bath for 6 hours, cooled then triturated with dry acetone. The acetone was decanted leaving a yellow solid which was partitioned between water (20 ml) and chloroform (20 ml) then basified with potassium carbonate. The organic layer was separated and the aqueous re-extracted with chloroform ( 2 x 20 ml). The combined organics were dried (potassium carbonate) then exaporated to dryness in vacuo to give a pale yellow solid which was purified by column chromatography on neutral alumina [Merck Art 1077, Grade 3, 100 g] using dichloromethane/methanol (20 : 1). The title compound free base was obtained as a colourless solid (0.18 g, 30%). The dihydrochloride salt had mp 173 - 175°C (propan-2-ol/diethyl ether). (Found: C, 42.08%; H, 6.86%; N, 12.80%; $C_{10}H_{15}N_3S.2HCl.0.4(CH_3)_2CHOH$ 0.75 $H_2O$ requires C, 42.06%; H, 6.84%; N, 13.14%); m/e 209 ($M^+$ of free base); $\delta$ (360 MHz, $D_2O$) 1.86 - 1.94 (2H, m, 5 and 8CH); 2.11(2H, dt, J = 3, 8 Hz, 5 and 8CH); 2.38 - 2.40 (1H, m, 4CH); 3.26 - 3.52 (6H, m, 2CH, 3CH, $6CH_2$ and $7CH_2$); 3.70 - 3.78 (1H, m, 2CH); 6.72 (1H, s, S-C$\underline{H}$=).

EXAMPLE 2

3-[4-(1,3-Thiazole)-yl]quinuclidine hydrogen oxalate

A solution of sodium nitrite (0.77 g, 11 mmol) in water (2 ml) was added dropwise to a cooled (-15°C), stirred solution of 3-[4-(2-amino-1,3-thiazol)-yl] quinuclidine (0.47 g, 2.2 mmol) in 50% hypophosphorus acid (10 ml), keeping the reaction mixture below -10°C. The mixture was allowed to warm to 6°C and left standing at this temperature for 5 days. After dilution with water (30 ml) the solution was basified with potassium carbonate and extracted into dichloromethane (5 x 30 ml). The combined organics were washed with water (50 ml), dried (sodium sulphate) then evaporated in vacuo to give a yellow oil which was purified by column chromatography on neutral alumina [Merck Art 1077, Grade 3, 35 gl using dichloromethane/methanol (60:1) to give the title compound free base as a pale yellow oil (70 mg). The hydrogen oxalate salt had mp 122-124°C (acetone/methanol (10:1)). (Found: C, 46.46%; H, 5.15%; N, 7.91%; $C_{10}H_{14}N_2S$. 1.75 $C_2H_2O_4$ requires C,46.08%; H, 5.01%; N, 7.96%) $\delta$ (360 MHz, $D_2O$) 1.83 - 1.90 and 2.07 - 2.20 (each 2H, each m, 5 and $8CH_2$); 2.46 - 2.50 (1H, m, 4CH); 3.30 - 3.46 (4H, m); 3.61 - 3.66 (1H, m); 3.72 - 3.78 (1H, m) and 3.81 - 3.88 (1H, m)($2CH_2$, 3CH, $6CH_2$ and $7CH_2$); 7.62 (1H, dd, J = 1.8, 2 Hz, S-C$\underline{H}$=C); 9.21 (1H, d, J = 2 Hz, N=C$\underline{H}$-S).

EXAMPLE 3

3-Hydroxy-3-[2-(4-methyl-1,3-oxazol)-yl] quinuclidine

A solution of n-butyllithium in hexane (1.6M, 50ml, 80mmol) was added dropwise over 30 min to a stirred solution of 4-methyloxazole (7.26g, 87.5mmol) in tetrahydrofuran (80ml) under nitrogen, keeping the temperature below -65°. After a further 30 min at -70°, a solution of quinuclidin-3-one (9.10g, 72.8mmol) in tetrahydrofuran (20ml) was added over 5 min. The mixture was kept at -70° for 2h, then warmed to 25° overnight. Saturated ammonium chloride solution (50ml) was added, the organic layer separated, and the aqueous layer extracted three times with dichloromethane. The combined organic layers were dried over potassium carbonate and evaporated. The residue was dissolved in boiling ethyl acetate (250ml), filtered and concentrated to 100ml. The product crystallised on cooling as colourless prisms (4.98g, 33%), m.p. 176-177°. (Found: C, 63.25%; H, 7.73%; N,13.40%. $C_{11}H_{16}N_2O_2$ requires: C, 63.44%; H, 7.74%; N, 13.45%) $\nu$ max: 3200-2500 (br),1600, 1550; m/e: 208 ($M^+$); $\delta$ ($CDCl_3$): 1.32-1.57 (3H, m, 5CH and $8CH_2$); 2.11-2.19 (2H, m, 4CH and 5CH); 2.17 (3H, d, J = 1Hz, $CH_3$); 2.76-2.88 (3H, m, $6CH_2$ and 7CH); 2.94-3.01 (1H, m, 7CH); 2.96 (1H, dd, J = 1Hz and 14Hz, 2CH); 3.78 (1H, dd, J = 2Hz and 14Hz, 2CH); 7.35 (1H, q, J = 1Hz, oxazole-H).

EXAMPLE 4

3-[2-(4-Methyl-1,3-oxazol)-yl]quinuclidine hydrochloride

a) 3-Chloro-3-[2-(4-methyl-1,3-oxazol)-yl] quinuclidine

3-Hydroxy-3-[2-(4-methyl-1,3-oxazol)-yl] quinuclidine from Example 3 (1.50g. 7.21mmol) was added portionwise to stirred, ice-cooled thionyl chloride (15ml), then the resulting solution heated under reflux for 1.5h. The cooled solution was evaporated, aqueous potassium carbonate (2M, 50ml) added and extracted three times with dichloromethane. Evaporation of the dried organic layers yielded the product as a brown oil (1.21g, 74%), which was used immediately in the next reaction. m/e 228, 226 ($M^+$ $^{37}Cl$, $^{35}Cl$); $\delta$ ($CDCl_3$): 1.22-1.30 (1H, m, 5CH); 1.54-1.67 (3H, m, 5CH and $8CH_2$); 2.19 (3H, d, J = 1Hz, $CH_3$); 2.64-2.66 (1H, m, 4CH); 2.72-2.76 (2H, m) and 2.90-3.06 (2H, m, $6CH_2$ and $7CH_2$); 3.43 (1H, d, J = 15Hz, 2CH); 4.30 (1H, dd, J = 2Hz and 15Hz, 2CH); 7.39 (1H, q, J = 1Hz, oxazole H).

b) 3-[2-(4-Methyl-1,3-oxazol)-yl]quinuclidine hydrochloride

A solution of the foregoing chloroquinuclidine (1.21g, 5.34mmol), tributyltin hydride (2.0ml, 7.4mmol) and azobisisobutyronitrile (AIBN) (50mg) was heated under reflux under a nitrogen atmosphere. Further portions of AIBN were added after 3h and 5h. After 8h the cooled solution was partitioned between 2M hydrochloric acid and dichloromethane. The organic layer was extracted with more hydrochloric acid, the combined acid layers basified with solid potassium carbonate and extracted four times with dichloromethane. Chromatography of the residue from evaporation of the organic layers on alumina, eluting with dichloromethane/1% methanol yielded the title product (265mg), which was converted to the hydrochloride (256mg), m.p. 217-219° (dec). (Found: C, 54.1%; H, 7.3%; N, 11.3%; Cl 18.4%; $C_{11}H_{16}N_2O.1.4HCl$ requires C, 54.3%; H, 7.2%; N, 11.5%; Cl, 20.4%) m/e: 192 ($M^+$); $\delta$ ($D_2O$),1.78-1.92 (3H, m, 5CH and $8CH_2$); 2.08-2.13 (1H, m, 5CH); 2.12 (3H, d, J = 1Hz, $CH_3$); 2.55-2.58 (1H, m, 4CH); 3.28-3.41 (4H, m, $6CH_2$ and $7CH_2$); 3.64-3.76 (3H, m, $2CH_2$ and 3CH); 7.58 (1H, q, J = 1Hz, oxazole H).

EXAMPLE 5

(+)-3-[2-(4-methyl-1,3-oxazol)-yl]quinuclidine hemi dibenzoyl tartrate

A solution of 3-[2-(4-methyl-1.3-oxazol)-yl] quinuclidine from Example 4 (679mg, 3.54mmol) in ethanol (1.0ml) was treated with (+)-O,O'dibenzoyl tartaric acid (317mg, 0.843mmol) in ethanol (1.5ml). The resulting solution was left at 4° for 24h, and the crystals formed collected and washed with cold ethanol (382mg). This was recrystallised from ethanol to yield the title compound (182mg), m.p. 164-165°, $[\alpha]_D$ +67.5°. A portion of this material (10mg), was converted to the borane complex by first liberating the free base, then treating with excess borane in tetrahydrofuran at 0° followed by aqueous work-up, and extraction into dichloromethane. The borane complex isolated from the organic layer was analysed by high performance liquid chromatography on a cyclobond I-acetylated column, in 40% methanol-0.4% triethylamine acetate, pH 4.0, which indicated an enantiomeric excess of 72%. (Found: C, 64.14%; H, 6.23%; N, 7.42%;

$C_{11}H_{16}N_2O$. 0.5 $C_{18}H_{14}O_8$.0.2$H_2O$ requires: C, 64.06%; H, 6.29%; N, 7.47%); m/e 192 ($M^+$); δ (360MHz, $D_2O$): 1.76-1.90 (2H, m, 5CH and 8CH); 2.04-2.16 (2H, m, 5CH and 8CH); 2.14 (3H, s, oxazole-$CH_3$); 2.54-2.58 (1H, m, 4CH); 3.29-3.43 (4H, m, 6$CH_2$ and 7$CH_2$); 3.63-3.76 (3H, m, 2$CH_2$ and 3CH); 5.72 (1H, s, $CHCO_2H$); 7.53-7.60 (3H, m, oxazole CH + phenyl 3CH and 5CH); 7.71 (1H, t. J = 6Hz, phenyl 4CH); 8.12 (2H, d. J = 6Hz, phenyl 2CH and 6CH).

EXAMPLE 6

(-)-3-[2-(4-Methyl-1,3-oxazol)-yl]quinuclidine hemi dibenzoyl tartrate

The mother liquors from the initial crystallisation in Example 5 were basified with aqueous potassium carbonate and extracted with dichloromethane. The organic extracts were dried over magnesium sulphate and evaporated to yield the free base (424mg, 2.21mmol). This material in ethanol (0.6ml) was treated with (-)O,O' dibenzoyl tartaric acid (313mg, 0.832mmol) in ethanol (1.5ml). The resulting solution was left at 4° for 18h and the crystals collected (286mg). This was recrystallised from ethanol to give the title compound (81mg), m.p. 162.5-164.5°, $[\alpha_D]$ -66.2°. A portion of this material (10mg) was converted to the borane complex and analysed as described in Example 5, indicating an enantiomeric excess of 92%. (Found: C, 63.84%; H, 6.26%; N, 7.21%; $C_{11}H_{16}N_2O$.0.5 $C_{18}H_{14}O_8$. 0.25$H_2O$ requires C, 63.90%; H, 6.30%; N, 7.45%); m/e 192 ($M^+$); δ (360MHz. $D_2O$); 1.76-1.88 (2H, m, 5CH and 8CH); 2.06-2.16 (2H, m, 5CH and 8CH); 2.13 (3H, d, J = 1Hz, oxazole $CH_3$); 2.54-2.58 (1H, m, 4CH); 3.30-3.43 (4H, m, 6$CH_2$ and 7$CH_2$); 3.63-3.76 (3H, m, 2$CH_2$ and 3CH); 5.72 (1H, s, $CHCO_2H$); 7.53-7.60 (3H, m, oxazole CH + phenyl 3CH and 5CH); 7.71 (1H, t, J = 6Hz, phenyl 4CH); 8.12 (2H, d, J = 6Hz, phenyl 2CH and 6CH).

EXAMPLE 7

3-Hydroxy-3-[2-(1,3-oxazol)-yl]quinuclidine

A solution of n-butyllithium in hexane (2.5M, 31ml, 77.5mmol) was added dropwise over 20 min to a stirred solution of oxazole (5.89g, 85.4mmol) in tetrahydrofuran (80ml) under nitrogen, keeping the temperature below -55°. After a further 30 min at -70°, a solution of quinuclidin-3-one (8.9g, 71.2mmol) in tetrahydrofuran (23ml) was added. The mixture was allowed to warm slowly to 20° overnight. Saturated ammonium chloride solution (50ml) was added, the organic layer separated and the aqueous layer extracted three times with dichloromethane. The combined organic layers were dried over potassium carbonate and evaporated. The residue was extracted with boiling ethyl acetate (250ml), filtered and concentrated to 80ml. On cooling the title compound was obtained as colourless needles (2.58g, 19%), m.p. 196-198°. (Found: C, 61.71%; H, 7.32%; N, 14.26%; $C_{10}H_{14}N_2O_2$ requires: C, 61.84%; H, 7.27%; N, 14.42%); ν max 3100-2500 (br), 1560; m/e (CI) 195 ($M+H^+$); δ (360MHz, $CDCl_3$) 1.27-1.37 (1H, m, 8CH); 1.45-1.57 (2H, m, 5CH and 8CH); 1.60-1.90 (1H, br, OH); 2.12-2.22 (2H, m, 4CH and 5CH); 2.78-2.92 (3H, m, 6CH and 7$CH_2$); 2.96-3.04 (1H, m, 6CH); 3.01 (1H, dd, J = 1Hz and 14.5Hz, 2CH); 3.84 (1H, dd, J = 2Hz and 14.5Hz, 2CH); 7.10 (1H, d, J = 1Hz, oxazole 4CH); 7.66 (1H, d, J = 1Hz, oxazole 5CH).

EXAMPLE 8

3-Hydroxy-3-[2-(4-ethyl-1,3-oxazol)-yl] quinuclidine

a) Ethyl 4-ethyloxazole-5-carboxylate

Ethyl 2-chloro-3-oxopentanoate (29.2g, 0.164mol) prepared by the method of E.A. Falco, P.B. Russell and G.H. Hitchings, J. Am. Chem. Soc., 1951, 73, 3753, and ammonium formate (60g, 0.95mol) were heated in 100% formic acid (180ml) under reflux for 5h. The cooled mixture was diluted with water (300ml) and diethyl ether (200ml), then neutralised with aqueous sodium hydroxide (5M). The layers were separated and the aqueous layer extracted with more ether (4 x 200ml). The combined ether layers were dried over magnesium sulphate and the solvent removed by distillation at atmospheric pressure. Vacuum distillation of the residue yielded the desired oxazole (11.2g, 40%) b.p. 100-106° (7mmHg). This was contaminated with starting material. An analytical sample, obtained by extracting into 5M sulphuric acid, had b.p. 97-98° (7mmHg). (Found C, 56.64%; H, 6.63%; N, 8.24%; $C_8H_{11}NO_3$ requires: C, 56.80%; H, 6.55%; N, 6.28%); m/e 169 ($M^+$): δ (360MHz, $CDCl_3$) 1.27 (3H, J = 7.5Hz, $ArCH_2CH_3$); 1.40 (3H, t. J = 7Hz, $CO_2CH_2CH_3$); 2.91 (2H, q, J = 7.5Hz, $ArCH_2CH_3$); 4.40 (2H, q, J = 7Hz, $CO_2CH_2CH_3$); 7.89 (1H, s, ArH).

b) 4-Ethyloxazole

A solution of the foregoing oxazole ester (23.2g, 0.14mol) in ethanol (25ml) and aqueous sodium hydroxide (2M, 75ml, 0.15mol) was stirred at 22° for 2h. The solvent was then evaporated and the residue dried under vacuum over phosphorus pentoxide. A suspension of the sodium salt in quinoline (42ml) containing quinoline sulphate (26.7g, 0.075mol) was heated in an oil bath at 230°. The oxazole was collected at 92-110° and dried over KOH pellets and redistilled, b.p. 98-102°, yield 3.73g (27%). (Found: C, 58.73%; H, 7.49%; N,13.41%; $C_5H_7NO$. $0.3H_2O$ requires C, 58.58%; H, 7.47%; N, 13.66%); δ (60MHz, $CDCl_3$) 1.25 (3H, t. J = 7Hz, $CH_2\underline{CH_3}$); 2.55 (2H, q, J = 7Hz, $\underline{CH_2}CH_3$); 7.25 (1H, s, 5CH); 7.70 (1H, s, 2CH).

c) 3-Hydroxy-3-[2-(4-ethyl-1,3-oxazol)-yl] quinuclidine

A solution of n-butyllithium in hexane (2.5M, 14ml, 35mmol) was added dropwise over 20 min to a stirred solution of the foregoing 4-ethyloxazole (3.73g, 38.5mmol) in dry tetrahydrofuran (40ml) under nitrogen at -75°. After a further 30 min, a solution of quinuclidin-3-one (4.01g, 32.1mmol) in tetrahydrofuran (10ml) was added and the mixture allowed to warm slowly to 20° overnight. Saturated aqueous ammonium chloride (50ml) was added and the layers separated. The aqueous layer was extracted with dichloromethane (4 x 50ml) and the combined organic layers dried over magnesium sulphate and evaporated. Column chromatography of the residue on neutral alumina, grade III, in dichloromethane/methanol (99:1) yielded the title compound (693mg, 10%), m.p. 118-120° (Found: C, 64.10%; H, 8.03%; N, 12.27%; $C_{12}H_{18}N_2O_2$.$0.1H_2O$ requires C, 64.32%; H, 8.19%; N. 12.50%); m/e 222 ($M^+$); δ (360MHz, $CDCl_3$) 1.22 (3H, t, J = 7.5Hz, $CH_2\underline{CH_3}$); 1.32-1.56 (3H, m, 5CH and $8CH_2$); 1.90-2.10 (1H, br, OH); 2.12-2.20 (2H, m, 4CH and 5CH); 2.55 (2H, dq, J = 1Hz and 7.5Hz, $\underline{CH_2}CH_3$); 2.73-2.89 (3H, m, $6CH_2$ and 7CH); 2.94-2.99 (1H, m, 7CH); 2.96 (1H, dd, J = 2Hz and 14Hz, 2CH); 3.78 (1H, dd, J = 2Hz and 14Hz, 2CH); 7.33 (1H, t, J = 1Hz, oxazole CH).

EXAMPLE 9

3-[2-(4-Ethyl-1,3-oxazol)-yl]quinuclidine hydrogen fumarate

a) 3-Chloro-3-[2-(4-ethyl-1,3-oxazol)-yl) quinuclidine

3-Hydroxy-3-[2-(4-ethyl-1,3-oxazol)-yl] quinuclidine from Example 8 (640mg, 2.88mmol) was added to ice cold thionyl chloride (6ml) and the resulting solution heated under reflux for 1.5h. The cooled solution was evaporated, residual thionyl chloride removed using a toluene azeotrope, and the residue partitioned between aqueous potassium carbonate (2M, 50ml) and dichloromethane (100ml). The organic layer was dried over magnesium sulphate to give the 3-chloroquinuclidine (718mg), δ (360MHz, $CDCl_3$) 1.24 (3H, t, J = 7.5Hz, $CH_2\underline{CH_3}$); 1.56-1.63 (3H, m, 5CH and $8CH_2$); 2.24-2.29 (1H, m, 5CH); 2.57 (2H, dq, J = 1Hz and 7.5Hz, $\underline{CH_2}CH_3$); 2.64-2.67 (1H, m, 4CH); 2.72-2.77 (2H, m, 6CH and 7CH); 2.92-2.97 (1H, m, 6CH); 3.02-3.07 (1H, m, 7CH); 3.44 (1H, d, J = 15Hz, 2CH); 4.32 (1H, dd, J = 2Hz and 15Hz, 2CH); 7.37 (1H, t, J = 1Hz, oxazole H).

b) 3-[2-(4-Ethyl-1,3-oxazol)-yl]quinuclidine hydrogen fumarate

A solution of the foregoing chloroquinuclidine (710mg, 2.85mmol), tributyltin hydride (1.1ml, 4.1mmol) and azobisisobutyronitrile (AIBN) (10mg) in dry tetrahydrofuran (12ml) under nitrogen was stirred and heated under reflux. After 1.5h, a further portion of AIBN was added and heating continued for a further 4h. The cooled solution was partitioned between 2M hydrochloric acid (30ml) and dichloromethane (40ml). The aqueous layer was basified with solid sodium carbonate and extracted with dichloromethane (4 x 60ml). The combined organic layers were dried over magnesium sulphate, evaporated and the residue purified by column chromatography on neutral alumina, grade III, in dichloromethane/methanol (99:1) to yield the title compound free base (368mg, 63%), which was converted to the hydrogen fumarate and recrystallised from propan-2-ol to yield the title compound (273mg). m.p. 108-113° (Found: C, 58.75%; H, 6.84%; N, 8.60;% $C_{12}H_{18}N_2O$.1.1 $C_4H_4O_4$ requires: C, 58.98%; H, 6.76%; N, 8.39%); m/e 206 ($M^+$); δ (360MHz, $D_2O$) 1.17 (3H, t. J = 7.5Hz, $CH_2CH_3$); 1.78-1.89 (2H, m, $8CH_2$); 2.07-2.15 (2H, m, $5CH_2$); 2.51 (2H, dq, J = 1Hz and 7.5Hz, $CH_2CH_3$); 2.54-2.58 (1H, m, 4CH); 3.32-3.41 (4H, m, $6CH_2$ and $7CH_2$); 3.67-3.78 (3H, m, $2CH_2$ and 3CH); 6.66 (2.2H, s, $HO_2C$ $\underline{CH}$=$\underline{C}HCO_2H$); 7.59 (1H, t, J = 1Hz, oxazole H).

EXAMPLE 10

3-Hydroxy-3-[2-(4-methyl-1,3-thiazol)-yl] quinuclidine

A solution of n-butyllithium in hexane (1.6H, 25ml, 40mmol) was added to a stirred solution of 4-methylthiazole in tetrahydrofuran (50ml) under nitrogen, keeping the temperature below -60°. After 1h at -60° a solution of quinuclidin-3-one (4.55g, 36.4mmol) in tetrahydrofuran was added dropwise, maintaining the same temperature. After a further 0.5h at -60°, the mixture was allowed to warm slowly to 20°. Methanol (10ml) was added and the solvent evaporated. The residue was partitioned between dichloromethane and water and the organic layer dried over sodium sulphate, and evaporated. Recrystallisation of the solid residue from ethyl acetate yielded the title compound (4.2g, 51%), m.p. 183-186°. (Found: C, 58.9%; H, 7.3%; N, 12.3%; $C_{11}H_{16}N_2OS$ requires: C, 58.9%; H, 7.2%; N, 12.5%). ν max (Nujol) 3200-2500 (br), 1530 $cm^{-1}$; m/e 224 ($M^+$); δ (360MHz, $CDCl_3$) 1.38-1.50 (2H, m, $8CH_2$); 1.56-1.65 (1H, m, 5CH); 2.04-2.07 (1H, m, 4CH); 2.15-2.24 (1H, m, 5CH); 2.42 (3H, d, J = 1Hz, Ar $CH_3$); 2.67-2.93 (4H, m, $6CH_2$ and $7CH_2$); 2.97 (1H, dd, J = 2Hz and 14Hz, 2CH); 3.71 (1H, dd, J = 2Hz and 14Hz, 2CH); 4.06-4.26 (1H, br, OH); 6.82 (1H, q, J = 1Hz, SC<u>H</u>=C).

EXAMPLE 11

3-[2-(4-Methyl-1,3-thiazol)-yl]-1-azabicyclo[2.2.2]oct-2-ene dihydrochloride

3-Hydroxy-3-[2-(4-methyl-1,3-thiazol)-yl] quinuclidine from Example 10 (1.0g, 4.5mmol) was added to stirred thionyl chloride (10ml) at 20° and the solution heated to reflux for 1.5h. The cooled solution was evaporated and last traces of thionyl chloride removed using a toluene azeotrope. The residue was dissolved in boiling ethanol, and cooled, whereupon the title compound crystallised (225mg, 18%) m.p. 179-182° (Found: C, 47.0%; H, 5.7%; N,10.0%; $C_{11}H_{14}N_2S.2HCl$ requires: C, 47.3%; H, 5.8%; N, 10.0%). ν max 2100-2500 (br), 1590; m/e 206 ($M^+$); δ (360MHz, $D_2O$) 1.88-1.99 (2H, m, 5CH and 8CH); 2.19-2.28 (2H, m, 5CH and 8CH); 2.52 (3H, d, J = 1Hz, $CH_3$); 3.24-3.33 (2H, m, 6CH and 7CH); 3.68-3.77 (3H, m, 4CH, 6CH and 7CH); 7.48 (1H, q, J = 1Hz, SC<u>H</u>=C); 7.52 (1H, d, J = 1Hz, NC<u>H</u>=C).

EXAMPLE 12

3-[2-(4-Methyl-1,3-thiazol)-yl]quinuclidine dihydrochloride

3-[2-(4-Methyl-1,3-thiazol)-yl]-1-azabicyclo [2.2.2]oct-2-ene dihydrochloride from Example 11 (150mg, 0.537mmol) was hydrogenated over sulphided platinum (150mg) in methanol (25ml) at 40 p.s.i. for 18h. The catalyst was removed by filtration through Hyflo and the solvent evaporated to dryness to yield the title compound as an amorphous hygroscopic solid (125mg, 83%); m/e 208.1036; $C_{11}H_{16}N_2S$ requires 208.1034; δ (360MHz, $D_2O$) 1.84-1.96 (2H, m, $8CH_2$); 2.07-2.15 (2H, m, $5CH_2$); 2.40 (3H, s, $CH_3$); 2.49-2.55 (1H, m, 4CH); 3.27-3.48 (4H, m, $6CH_2$ and $7CH_2$); 3.74-3.94 (3H, m, $2CH_2$ and 3CH); 7.15 (1H, s, SC<u>H</u>=C).

EXAMPLE 13

3-[2-(5-Methyl-1,3-oxazol)-yl]quinuclidine hydrogen fumarate

Quinuclidine-3-carboxylic acid hydrochloride, prepared by the method of C.A. Grob and E. Renk, Helv. Chim. Acta, 1954, <u>37</u>, 1689 (4.88g, 25.5mmol) was added to stirred thionyl chloride (60ml) with ice cooling, and the resulting solution was heated to reflux for 2.5h. The cooled mixture was evaporated and last traces of thionyl chloride removed using a toluene azeotrope. The residue was suspended in dry dichloromethane (50ml) under nitrogen, propargyl amine (3.5ml, 51 mmol) and triethyl amine (6.0ml, 43mmol) added and the mixture stirred at 20° for 16h. Aqueous potassium carbonate (2M, 30ml) was added and solution extracted with dichloromethane (4 x 50ml). The combined extracts were dried over potassium carbonate and evaporated to yield the crude propargyl amide (2.8g). This material was dissolved in acetic acid (45ml), mercuric acetate (262mg, 0.82mmol) added and the mixture heated under reflux for 3h. The cooled solution was evaporated and aqueous potassium carbonate added. The product was extracted into dichloromethane (4 x 50ml), dried over potassium carbonate and evaporated to give a gum which was purified by column chromatography on neutral alumina, grade III, eluting with dichloromethane/methanol (99:1) to give the title compound free base as a colourless gum (748mg, 16% overall). The hydrogen fumarate salt had m.p. 123-125° (propan-2-ol/diethyl ether). (Found: C, 57.85%; H, 6.52%; N, 8.95%; $C_{11}H_{16}N_2O$. 1.1 $C_4H_4O_4$ requires: C, 57.81%; H, 6.43%; N, 8.76%); m/e 192 ($M^+$); δ (360MHz, $D_2O$) 1.78-1.89 (2H, m, 8$CH_2$); 2.07-2.15 (2H, m, 5$CH_2$); 2.29 (3H, d, J = 1Hz, $CH_3$); 2.54-2.57 (1H, m, 4CH); 3.31-3.42 (4H, m, 6$CH_2$ and 7$CH_2$); 3.62-3.76 (3H, m, 2$CH_2$ and 3CH); 6.66 (2.2H, s, $HO_2CC\underline{H}=C\underline{H}CO_2H$); 6.76 (1H, q, J = 1Hz, $NC\underline{H}=CO$).

EXAMPLE 14

1-Methyl-3-[2-(5-methyl-1,3-oxazol)-yl]piperidine hydrogen oxalate

a) 1-Methyl-N-(3-propynyl)piperidine-3-carboxamide

1-Methyl piperidine-3-carboxylic acid hydrochloride (21g, 0.12mol) was added to stirred thionyl chloride (50ml) at 20° and the mixture heated under reflux for 15min. The cooled solution was evaporated and residual thionyl chloride removed using a toluene azeotrope. This material was suspended in dry tetrahydrofuran (250ml) under nitrogen, cooled to 0° and treated with propargyl amine (6.45g, 0.12mol) and triethylamine (12.1g, 0.12mol). The cooling bath was removed and the mixture stirred at 20° for 18h. The solvent was evaporated, the residue dissolved in water, basified to pH 10 with potassium carbonate and extracted three times with dichloromethane. The extracts were dried over sodium sulphate and evaporated to yield the propargyl amide (17g, 81%), which was characterised as the hydrogen oxalate salt, m.p. 150°. (Found: C, 53.0%; H. 6.8%; N, 10.4%; $C_{10}H_{16}N_2O$. $C_2H_2O_4$ requires: C, 53.3%; H, 6.7%; N, 10.0%); ν max (Nujol) 3250, 3190, 3080, 2120(w), 1640; m/e 180 ($M^+$); δ (360MHz, $D_2O$) 1.54-1.66 (1H, m, 5CH); 1.75-2.10 (4H, m, 4$CH_2$, 5CH and NH); 2.59-2.62 (1H, m, $C\equiv C\underline{H}$); 2.73-2.84 (1H, m, 3CH); 2.89 (3H, s, $NCH_3$); 2.91-3.07 (2H, m, 2CH and 6CH); 3.42-3.64 (2H, m, 2CH and 6CH); 3.95-3.98 (2H, m, $HNC\underline{H_2}C\equiv$).

b) 1-Methyl-3-[2-(5-methyl-1,3-oxazol)-yl] piperidine hydrogen oxalate

A solution of the foregoing propargyl amide free base (1.8g, 10mmol) in acetic acid (20ml) containing mercuric acetate (30mg, 0.1mmol) was heated under reflux for 5h, cooled, and evaporated. The residue was partitioned between dichloromethane and saturated potassium carbonate, the organic layer dried over sodium sulphate and evaporated. The resulting oil was purified by chromatography on neutral alumina, grade III in ethyl acetate. The product was treated with ethereal oxalic acid and crystallised from propan-2-ol to yield the title compound (680mg, 25%), m.p. 101-103°. (Found: C, 51.1%; H, 6.4%; N, 9.5%; $C_{10}H_{16}N_2O.C_2H_2O_4$.0.67 $H_2O$ requires: C, 51.1%; H, 6.8%; N, 9.9%). δ (360MHz, DMSO) 1.53-1.65 (1H, m, 5CH); 1.76-1.91 (2H, m, 5CH and 4CH); 2.05-2.11 (1H, m, 4CH); 2.27 (3H, d, J = 1Hz, oxazole $CH_3$); 2.76 (3H, s, $NCH_3$); 2.86-2.95 (1H, m, 6CH); 3.09 (1H, t, J = 12Hz, 2CH); 3.28-3.36 (2H, m, 3CH and 6CH); 3.61 (1H, d, J = 12Hz, 2CH); 6.77 (1H, q, J = 1Hz, oxazole CH).

EXAMPLE 15

1-Methyl-3-[2-(5-methyl-1,3-oxazol)-yl]-1,2,5,6-tetrahydropyridine sesquifumarate

1-Methyl-1,2,5,6-tetrahydropyridine carboxylic acid hydrochloride, obtained by hydrolysis of arecoline with 5M hydrochloric acid (5.07g, 36mmol) was added to stirred thionyl chloride (50ml) at 20° and the resulting solution heated under reflux for 2.5h. The cooled solution was evaporated and residual thionyl chloride removed using a toluene azeotrope. The residue was suspended in dry dichloromethane (50ml) under nitrogen, cooled in ice and treated with propargyl amine (3.6ml, 53mmol). The cooling bath was removed and the mixture stirred at 20° for 18h. Aqueous potassium carbonate (2M, 40ml) was added and the product extracted with dichloromethane (4 x 60ml). The extracts were dried over magnesium sulphate and evaporated to give the crude propargyl amide (5.49g). This material was dissolved in acetic acid (70ml), mercuric acetate (0.53g, 1.7mmol) added, and the mixture heated under reflux for 3h. The cooled solution was evaporated to dryness, aqueous potassium carbonate (50ml) added and extracted with dichloromethane (3 x 50ml). The extracts were dried over magnesium sulphate and evaporated to yield a dark gum which was purified by column chromatography on silica in dichloromethane/methanol (95:5) to give the title compound free base (730mg, 11% overall), which was treated with fumaric acid (478mg, 4.12mmol) and recrystallised from ethanol to give the title compound (385mg), m.p. 145-147°. (Found: C, 54.45%; H, 5.72%; N, 7.94%; $C_{10}H_{14}N_2O$.1.5 $C_4H_4O_4$ requires: C, 54.54%; H, 5.72%; N, 7.95%; m/e 178 ($M^+$); δ (360MHz, $D_2O$) 2.32 (3H, d, J = 1 Hz, oxazole $CH_3$); 2.68-2.81 (2H, m, 5$CH_2$); 3.05 (3H, s, $NCH_3$);

3.26-3.34 (1H, m, 6CH); 3.62-3.67 (1H, m, 6CH); 3.95 (1H, dq, J = 2Hz and 16Hz, 2CH); 4.32 (1H, d, J = 16Hz, 2CH); 6.72 (3H, s, $HO_2CCH=CHCO_2H$); 6.82 (1H, q, J = 1Hz, NCH=C); 6.88-6.90 (1H, m, 4CH).

EXAMPLE 16

3-[2-(5-Methyl-1,3-oxazol)-yl]-1,2,5,6-tetrahydropyridine hydrochloride

a) 1-Ethenyloxycarbonyl-3-[2-(5-methyl-1,3-oxazol)-yl]-1,2,5,6-tetrahydropyridine

To a stirred solution of 1-methyl-3-[2-(5-methyl-1,3-oxazol)-yl]-1,2,5,6-tetrahydropyridine (550mg, 3.09mmol), from Example 15, in 1,2-dichloroethane (5ml) at -5° was added vinyl chloroformate (0.34ml, 3.7mmol). After 10 min, the solution was heated to reflux for 1.5h, then cooled, diluted with dichloromethane (20ml) and washed in turn with 0.5M hydrochloric acid (10ml) and saturated sodium hydrogen carbonate (10ml). The organic layer was dried over magnesium sulphate, evaporated and the residue in dichloromethane/ethyl acetate (95:5) filtered through a plug of silica gel to remove baseline material. Evaporation of the eluate yielded the title carbamate as an oil (450mg, 62%). m/e 234 ($M^+$); δ (360MHz, $CDCl_3$) 2.33 (3H, s, $CH_3$); 2.38-2.42 (2H, m, $5CH_2$); 3.62-3.68 (2H, m, $6CH_2$); 4.42-4.47 (2H, m, $2CH_2$); 4.47 (1H, dd, J = 2Hz and 6Hz, $CO_2CH=CH$, trans to O); 4.85 (1H, d, J = 14Hz, $CO_2CH=CH$, cis to O); 6.74 (1H, s, oxazole H); 6.74-6.78 (1H, m, 4CH); 7.24 (1H, dd, J = 6Hz and 14Hz, $CO_2CH=C$).

b) 3-[2-(5-Methyl-1,3-oxazol)-yl]-1,2,5,6-tetrahydropyridine hydrochloride

A solution of the foregoing vinyl carbamate (220mg, 0.94mmol) in methanolic hydrogen chloride (10ml) was stirred at 20° for 36h and evaporated to dryness. The residue was triturated with diethyl ether and recrystallised from propan-2-ol to yield the title compound, (132mg, 67%), m.p. 169-171° (Found: C, 52.74%; H, 6.62%; N,13.18%; $C_9H_{12}N_2O$. 1.15 HCl.0.04 $C_3H_8O$ requires: C, 52.53%; H, 6.51%; N, 13.43%); m/e 164 ($M^+$); δ (360MHz, $D_2O$) 2.32 (3H, d, J = 1Hz, $CH_3$); 2.62-2.68 (2H, m, $5CH_2$); 3.42 (2H, t, J = 6Hz, $6CH_2$); 4.06-4.09 (2H, m, $2CH_2$); 6.82 (1H, q, J = 1Hz oxazole CH); 6.88-6.92 (2H, m, 4CH).

EXAMPLE 17

1-Methyl-3-[4-(2-methyl-1,3-thiazol)-yl]-1,2,5,6-tetrahydropyridine hemifumarate

a) 1-Methyl-3-[4-(2-methyl-1,3,-thiazol)-yl] pyridinium iodide

3-[4-(2-Methyl-1,3-thiazol)-yl]pyridine hydrobromide (29.2g, 11.4mmol), prepared by the method of D.J. Brown et al, Aust. J. Chem.. 1980, 33, 2291, was partitioned between dichloromethane and aqueous potassium carbonate, and the organic layer dried over magnesium sulphate and evaporated to yield the free base (20.1g, 100%). This material was dissolved in acetone (100ml) with warming, cooled to 20° and methyl iodide (8.3ml, 13.3mmol) added with stirring. After 3 days at 20° the precipitate was collected, washed with acetone and ether and dried to yield the title compound (34.3g, 95%), m.p. 148-149° (Found: C, 37.58%; H, 3.50%; N, 8.78%; S, 10.04%; $C_{10}H_{11}IN_2S$ requires: C, 37.75%; H, 3.48%; N, 8.80%; S, 10.08%); m/e 191 ($M^+$); δ (360MHz, $D_2O$) 2.81 (3H, s, $SCCH_3$); 4.67 (3H, s, $NCH_3$); 8.07 (1H, s, SCH); 8.11 (1H, dd, J = 7Hz and 8Hz, 5CH); 8.73 (1H, d, J = 7Hz, 4CH); 8.88 (1H, d, J = 8Hz, 6CH); 9.26 (1H, s, 2CH).

b) 1-Methyl-3-[4-(2-methyl-1,3-thiazol)-yl]-1,2,5,6-tetrahydropyridine hemifumarate

A stirred solution of the foregoing pyridinium salt (19.1g, 60.1mmol) in water (150ml) and ethanol (150ml) was cooled in ice and sodium borohydride (2.30g, 60mmol) added. After the initial reaction, the cooling bath was removed and stirring continued for 2h at 20°. Excess borohydride was destroyed by adding concentrated hydrochloric acid dropwise, then the solution was basified with aqueous potassium carbonate and ethyl acetate (300ml) added. The layers were separated and the aqueous layer extracted with dichloromethane (3 x 200ml). The combined organic layers were dried over magnesium sulphate, evaporated and purified by column chromatography on silica in dichloromethane/methanol (95:5) to give the free base of the title compound (9.75g, 84%). A portion of this material (1.5g, 8.14mmol) was treated with fumaric acid (0.425g, 3.66mmol) in methanol and concentrated to yield the title hemifumarate (1.53g, 76% recovery), m.p. 139-141° (Found: C, 57.03%; H, 6.39%; N, 11.14%; $C_{10}H_{14}N_2S$. 0.5 $C_4H_4O_4$ requires: C,

57.12%; H, 6.39%; N, 11.10%); m/e 194 ($M^+$); δ (360MHz, DMSO) 2.28-2.33 (2H, m, $5CH_2$); 2.41 (3H, s, $SCCH_3$); 2.57-2.61 (2H, m, $6CH_2$); 3.30-3.32 (2H, m, $2CH_2$); 6.54 (1H, s, SCH); 6.54-6.57 (1H, m, 4CH); 7.29 (1H, s, $HO_2CC\underline{H}=C\underline{H}CO_2H$).

EXAMPLE 18

3-[4-(2-Methyl-1,3-thiazol)-yl]-1,2,5,6-tetrahydropyridine dihydrochloride

a) 1-Ethenyloxycarbonyl-3-[4-(2-methyl-1,3-thiazol)-yl]-1,2,5,6-tetrahydropyridine

A solution of 1-methyl-3-[4-(2-methyl-1,3-thiazol)-yl]-1,2,5,6-tetrahydropyridine (7.31g, 37.7mmol), from Example 17, in 1,2-dichloroethane (50ml), was treated with vinyl chloroformate (4.3ml, 47.1mmol) exactly as described in Example 16 to yield the title compound as a solid (7.10g, 75%). m/e (CI) 251 ($M+H^+$); δ - ($CDCl_3$) 2.35-2.41 (2H, m, $5CH_2$); 2.71 (3H, s, $CH_3$): 2.62-2.68 (2H, m, $6CH_2$); 4.36 (2H, brd, J = 12Hz, $2CH_2$); 4.48 (1H, dd, J = 1.5Hz and 6Hz, $CO_2CH=CH$, trans to O); 4.82 (1H, dd, J = 1.5Hz and 14Hz, $CO_2CH=CH$, cis to O); 6.75-6.82 (1H, m, 4CH); 6.89 (1H, s, thiazole CH); 7.26 (1H, dd, J = 6Hz and 14Hz, $CO_2C\underline{H}=CH_2$).

b) 3-[4-(2-Methyl-1,3-thiazol)-yl]-1,2,5,6-tetrahydropyridine dihydrochloride

A suspension of the foregoing vinyl carbamate (7.76g, 31.0 mmol) in methanolic hydrogen chloride was stirred at 20° for 36h and evaporated to dryness. The residue was triturated with diethyl ether and recrystallised from methanol to yield the title compound as fine needles (5.89g, 75%), m.p. 227-230° (dec). (Found: C, 42.3%; H, 5.6%; N, 11.0%; Cl, 28.0%; $C_9H_{12}N_2S.2HCl$ requires: C, 42.7%; H, 5.6%; N, 11.1%; Cl, 28.0%); m/e 180 ($M^+$); δ (360MHz, $D_2O$) 2.62-2.68 (2H, m, $5CH_2$); 2.88 (3H, s, $CH_3$); 3.43 (2H, t, J = 6Hz, $6CH_2$); 4.08-4.10 (2H, m, $2CH_2$); 6.70-6.72 (1H, m, 4CH); 7.54 (1H, s, thiazole CH).

EXAMPLE 19

1-Methyl-3-[4-(2-methyl-1,3-oxazol)-yl]-1,2,5,6-tetrahydropyridine hydrogen fumarate

a) 3-[4-(2-Methyl-1,3-oxazol)-yl]pyridine

A solution of 3-(bromoacetyl)pyridine hydrobromide, (28.1g, 0.10mol), prepared by the method of A. Dornow, H. Machens, K. Bruncken, Chem. Ber., 1951, 84, 148, and acetamide (12g, 0.20mol) in acetic acid (100ml) was heated under reflux for 22h, cooled, and the solvent evaporated. The residue was dissolved in water (100ml), dichloromethane (100ml) added and the solution basified with aqueous potassium carbonate. The mixture was filtered through Hyflo to remove a considerable amount of tar, and the layers separated. The aqueous layer was extracted with more dichloromethane (3 x 100ml) and the combined organic layers dried over magnesium sulphate and evaporated. Column chromatography of the residue on silica in dichloromethane/methanol (98:2), increasing to (95:5) yielded the desired oxazole as the second-eluted component as a pale orange solid (2.75g, 17%). An analytical sample was recrystallised from ether, m.p. 84-86° (Found: C, 67.40%; H, 5.09%; N, 17.41%; $C_9H_8N_2O$ requires: C, 67.49%; H, 5.03%; N, 17.49%); m/e 160 ($M^+$); δ (360MHz, $CDCl_3$) 2.54 (3H, s, $CH_3$); 7.33 (1H, dd, J = 5Hz and 8Hz, 5CH); 7.89 (1H, s, oxazole H); 8.01 (1H, dt, J = 1Hz and 8Hz, 4CH); 8.54 (1H, dd, J = 1Hz and 5Hz, 6CH); 8.93 (1H, dd, J = 1Hz, 2CH).

b) 1-Methyl-3-[4-(2-methyl-1,3-oxazol)-yl] pyridinium iodide

A solution of the foregoing pyridine (3.16g, 19.8mmol) and methyl iodide (1.5ml, 23.7mmol) in acetone (20ml) was stirred at 20° for 3 days. The precipitate was collected, washed with cold acetone and dried to yield the title compound as pale orange crystals, (5.12g, 86%), m.p. 180-182° (dec). (Found: C, 39.71%; H, 3.68%; N, 9.21%; $C_{10}H_{11}IN_2O$ requires: C, 39.76%; H, 3.67%; N, 9.27%); δ (360MHz, $D_2O$) 2.58 (3H, s, oxazole $CH_3$); 4.49 (3H, s, $NCH_3$); 8.10 (1H, dd, J = 6Hz and 8Hz, 5CH; 8.43 (1H, s, oxazole CH); 8.72-8.78 (2H, m, 4CH and 6CH); 9.11 (1H, s, 2CH).

c) 1-Methyl-3-[4-(2-methyl-1,3-oxazol)-yl]-1,2,5,6-tetrahydropyridine hydrogen fumarate

A solution of the foregoing pyridinium salt (5.54g, 18.3mmol) in water (50ml) and ethanol (50ml) was treated with sodium borohydride (0.70g, 18.4mmol) exactly as described in Example 17, to yield the title compound free base after chromatography as an oil (2.25g, 69%). A portion of this material (0883g, 4.96mmol) was treated with fumaric acid (0.546g, 4.71mmol) in methanol, concentrated and cooled to yield the title compound as colourless needles (833mg), m.p. 144-146° (Found: C, 56.92%; H, 6.23%; N, 9.36%; $C_{10}H_{14}N_2O.C_4H_4O_4$ requires: C, 57.14%; H, 6.17%; N, 9.52%); m/e 178 ($M^+$); δ (360MHz, $D_2O$); 2.44 (3H, s. oxazole $CH_3$); 2.62-2.72 (2H, m, $5CH_2$); 3.02 (3H, s, $NCH_3$); 3.24-3.32 (1H, m, 6CH); 3.58-3.65 (1H, m, 6CH); 3.89 (1H, dq, J = 1Hz and 16Hz, 2CH); 4.10 (1H, d, J = 16Hz, 2CH); 6.45-6.49 (1H, m, 4CH); 6.65 (2H, s, $HO_2C\underline{C}H=\underline{C}HCO_2H$); 7.73 (1H, s, oxazole CH).

## EXAMPLE 20

### 3-[4-(2-Methyl-1,3-oxazol)-yl]-1,2,5,6-tetrahydropyridine hydrogen oxalate

a) 1-Ethenyloxycarbonyl-3-[4-(2-methyl-1,3-oxazol)-yl]-1,2,5,6-tetrahydropyridine

A solution of 1-methyl-3-[4-(2-methyl-1,3-oxazol)-yl]-1,2,5,6-tetrahydropyridine (1.91g, 10.7mmol), from Example 19 in 1,2-dichloroethane (15ml) was treated with vinyl chloroformate (1.3ml, 13.9mmol) exactly as described in Example 16 to yield the title compound as an oil (1.54g, 61%). (Found: $M^+$, 234.1010; $C_{12}H_{14}N_2O_3$ requires 234.1004); δ (360MHz, $CDCl_3$) 2.33-2.38 (2H, m, $5CH_2$); 2.46 (3H, s, $CH_3$); 3.62-3.68 (2H, m, $6CH_2$); 4.18-4.23 (2H, m, $2CH_2$); 4.48 (1H, dd, J = 2Hz and 6Hz, $CO_2CH=CH$, trans to O); 4.82 (1H, d, J = 14Hz $CO_2CH=CH$, cis to O); 6.53-6.57 (1H, m, 4CH); 7.25 (1H, dd, J 6Hz and 14Hz, $CO_2\underline{CH}=CH_2$); 7.44 (1H, s, oxazole CH).

b) 3-[4-(2-Methyl-1,3,-oxazol)-yl]-1,2,5,6-tetrahydropyridine hydrogen oxalate

A solution of the foregoing carbamate (1.42g, 6.07mol) in methanolic hydrogen chloride (50ml) was stirred at 20° for 6h and evaporated to dryness. The residue was partitioned between aqueous potassium carbonate and dichloromethane, the organic layer dried over magnesium sulphate and evaporated. Column chromatography of the residue on grade III alumina in dichloromethane/methanol (99:1) yielded the title compound free base (755mg, 76%), which was converted to the hydrogen oxalate salt and recrystallised from methanol (972mg, 63% yield). m.p. 204-206° (Found: C, 49.80%; H. 5.65%; N, 10.52%; $C_9H_{12}N_2O.C_2H_2O_4.0.6H_2O$ requires: C, 49.85%; H, 5.78%; N, 10.57%); m/e 164 ($M^+$); δ (360MHz, $D_2O$) 2.45 (3H, s, $CH_3$); 2.54-2.60 (2H, m, $5CH_2$); 3.39 (2H, t, J = 6Hz, $6CH_2$); 3.93 (2H, d, J = 2Hz, $2CH_2$); 6.46-6.49 (1H, m, 4CH); 7.73 (1H, s, oxazole CH).

## EXAMPLE 21

### 1-Methyl-3-[2-(4-methyl-1,3-oxazol)-yl]-1,2,5,6-tetrahydropyridine sesquifumarate

a) 3-[2-(4-Methyl-1,3-oxazol)-yl]pyridine

A mixture of 3-cyanopyridine (31.2g, 0.30mol) and freshly distilled propargyl alcohol (84g, 1.50mol) was added dropwise with stirring to concentrated sulphuric acid (70ml), cooled in ice/salt, over 2.5h, keeping the temperature below 5°. When the addition was complete the mixture was allowed to warm to 20° over several hours, and stirred for 8 days. The dark mixture was poured into ice/water (1000 ml), filtered through Hyflo and washed with ethyl acetate (500ml). The aqueous layer was basified by cautious addition of a solution of sodium hydroxide (105g, 2.63mol) in water (250ml), extracted with dichloromethane (4 x 500ml), the combined extracts dried over magnesium sulphate and evaporated. Column chromatography of the residue on silica in dichloromethane/methanol (98:2) gave unreacted cyanopyridine as the first component; increasing to 95:5 yielded the desired oxazole (1.76g, 3.7%) as a crystalline solid. An analytical sample was recrystallised from hexane, m.p. 39-42° (Found: C, 66.69%; H, 5.18%; N, 17.14%; $C_9H_8N_2O.0.1H_2O$ requires: C, 66.74%; H, 5.10%; N, 17.30%); m/e 160 ($M^+$); δ (360MHz, $CDCl_3$) 2.26 (3H, d, J = 1Hz, $CH_3$); 7.38 (1H, ddd, J = 1Hz, 5Hz and 8Hz, 5CH); 7.48 (1H, q, J = 1Hz, oxazole CH); 8.28 (1H, dt, J = 1Hz and 8Hz, 8.67 (1H. dd, J = 1Hz and 5Hz, 6CH); 9.25 (1H, t, J 1Hz, 2CH).

b) 1-Methyl-3-[2-(4-methyl-1,3-oxazol)-yl pyridinium iodide

Methyl iodide (1.2ml, 18.9mmol) was added to a stirred solution of the foregoing pyridine (2.52g, 15.8mmol) in acetone (16ml). After 4 days at 20° the precipitate was collected, washed with acetone and dried to yield the title compound as a pale yellow powder (3.71g, 78%), m.p. 207-209° (dec) (Found: C, 39.94%; H, 3.72%; N, 9.05%; $C_{10}H_{11}IN_2O$ requires: C, 39.76%; H, 3.67%; N, 9.27%); δ (360MHz, $D_2O$) 2.28 (3H, d, J = 1Hz, oxazole $CH_3$); 4.52 (3H, s, $NCH_3$); 7.87 (1H, q, J = 1Hz, oxazole CH); 8.21 (1H, dd, J = 6Hz and 8Hz, 5CH); 8.90 (1H, d, J = 6Hz, 4CH); 8.98 (1H, d, J = 8Hz, 6CH); 9.39 (1H, s, 2CH).

c) 1-Methyl-3-[2-(4-methyl-1,3-oxazol)-yl]-1,2,5,6-tetrahydropyridine sesquifumarate

A solution of the foregoing pyridinium salt (4.21g, 13.9mmol) in water (40ml) and ethanol (40ml) was treated with sodium borohydride (0.53g, 13.9mmol) exactly as described in Example 17 to yield the title compound free base after chromatography (1.48g, 8.31mmol, 60%), which was treated with fumaric acid (0.940g, 8.10mmol) in methanol and concentrated to yield the sesquifumarate salt (940mg), m.p. 145-146° (Found: C, 54.36%; H, 5.74%; N, 7.89%; $C_{10}H_{14}N_2O$. 1.5 $C_4H_4O_4$ requires: C, 54.54%; H, 5.72%; N, 7.95%); m/e 178 ($M^+$); δ (360MHz, $D_2O$) 2.13 (3H, d, J = 1Hz, oxazole $CH_3$); 2.71-2.77 (2H, m, $5CH_2$); 3.06 (3H, s, $NCH_3$); 3.26-3.44 (1H, m, 6CH); 3.61-3.69 (1H, m, 6CH); 3.93-4.00 (1H, m, 2CH); 4.32 (1H, d, J = 16Hz, 2CH); 6.71 (3H, s, $HO_2C\underline{CH}=\underline{CH}CO_2H$); 6.90-6.96 (1H, m, 4CH); 7.56 (1H, q, J = 1Hz, oxazole CH).

EXAMPLE 22

1-Methyl-3-[5-(2-methyl-1,3-oxazole)-yl]-1,2,5,6-tetrahydropyridine sesquifumarate

a) 3-[5-(2-Methyl-1,3-oxazol)-yl]pyridine

Crude 3-(aminoacetyl)pyridine dihydrochloride (10.0g, 47.8mmol), prepared by the method of G.R. Clemo et al., J. Chem. Soc., 1938, 753, was suspended in triethyl orthoacetate (120ml) and heated under reflux in an oil bath at 140° for 4h. The cooled solution was decanted from some undissolved tar and evaporated. The residue was dissolved in water (100ml), stirred for 10 min then basified with solid sodium carbonate and extracted with dichloromethane (4 x 100ml). The combined extracts were dried over magnesium sulphate, evaporated and purified by column chromatography on silica in dichloromethane/methanol (98:2) to yield the title oxazole which crystallised on standing (5.33g, 69%), m.p. 29-33° (Found: C, 66.7%; H, 5.3%; N, 16.9%; $C_9H_8N_2O.0.15H_2O$ requires: C, 66.4%; H, 5.1%; N, 17.2%); m/e 160 ($M^+$); δ (250MHz, $CDCl_3$) 2.56 (3H, s, $CH_3$): 7.30 (1H, s, oxazole CH); 7.34 (1H, ddd, J = 1Hz, 5Hz and 8Hz, 5CH); 7.88 (1H, dt, J = 1Hz and 8Hz, 4CH); 8.54 (1H, dd, J = 2Hz and 5Hz, 6CH); 8.88 (1H, dd, J = 2Hz and 1Hz, 2CH).

b) 1-Methyl-3-[5-(2-methyl-1,3-oxazol)-yl] pyridinium iodide

Methyl iodide (2.2ml, 35.6mmol) was added to a stirred solution of the foregoing pyridine (4.75g, 29.7mmol) in acetone (30ml). After 4 days at 20° the precipitate was collected, washed with acetone and dried to give the title compound as off-white plates (7.06g, 79%), m.p. 189-191° (Found: C, 39.67%; H, 3.69%; N, 9.22%; $C_{10}H_{11}IN_2O$ requires: C, 39.76%; H, 3.67%; N, 9.27%); δ (360MHz, $D_2O$) 2.60 (3H, s, oxazole $CH_3$); 4.45 (3H, s, $NCH_3$); 7.73 (1H, s, oxazole CH); 8.10 (1H, dd, J = 6Hz and 7Hz, 5CH); 8.78-8.86 (2H, m, 4CH and 6CH); 9.16 (1H, s, 2CH).

c) 1-Methyl-3-[5-(2-methyl-1,3-oxazol)-yl]-1,2,5,6-tetrahydropyridine sesquifumarate

A solution of the foregoing pyridinium salt (6.96g, 23.0mmol) in water (65ml) and ethanol (65ml) was treated with sodium borohydride (0.88g, 23mmol), exactly as described in Example 17 to yield the title compound free base after chromatography (2.90g, 16.3mmol, 71%). To this was added fumaric acid (1.85g, 15.9mmol) in methanol (40ml), the solvent evaporated, the residue triturated with ether and recrystallised from propan-2-ol to give the sesquifumarate (1.74g), m.p. 140-142° (Found: C, 54.37%; H, 5.79%; N, 7.90%; $C_{10}H_{14}N_2O.1.5\ C_4H_4O_4$ requires: C, 54.54%; H, 5.72%; N, 7.95%); m/e 178 ($M^+$): δ (360MHz, $D_2O$) 2.45 (3H, s, oxazole $CH_3$); 2.62-2.74 (2H, m, $5CH_2$): 3.03 (3H, s, $NCH_3$); 3.24-3.33 (1H, m, 6CH); 3.60-3.68 (1H, m, 6CH); 3.90 (1H, dq, J = 1Hz and 16Hz, 2CH); 4.16 (1H, d, J = 16Hz, 2CH); 6.44-6.49 (1H, m, 4CH); 6.71 (3H, s, $HO_2C\underline{CH}=\underline{CH}CO_2H$); 6.94 (1H, s, oxazole CH).

EXAMPLE 23

3-[2-(4-Methyl-1,3-oxanol)-yl]-1,2,5,6-tetrahydropyridine hydrogen oxalate

a) 1-Ethenyloxycarbonyl-3-[2-(4-methyl-1,3-oxanol)-yl]-1,2,5,6-tetrahydropyridine

A solution of 1-methyl-3-[2-(4-methyl-1,3-oxazol)-yl]-1,2,5,6-tetrahydropyridine (1.20g, 6.74mmol), from Example 21, in 1,2-dichloroethane (10ml) was treated with vinyl chloroformate (0.80ml, 8.8mmol) exactly as described in Example 16 to yield the title compound as a waxy solid (1.11g, 70%) δ (360MHz, $CDCl_3$) 2.19 (3H, s, $CH_3$); 2.34-2.41 (2H, m, $5CH_2$); 3.60-3.69 (2H, m, $6CH_2$); 4.43-4.51 (3H, m, $2CH_2$ and $CO_2CH=CH$, trans to O); 4.86 (1H, d, J = 14Hz, $CO_2CH=CH$, cis to O); 6.80-6.85 (1H, m, 4CH); 7.23 (1H, dd, J = 6Hz and 14Hz, $CO_2CH=CH_2$); 7.31 (1H, s, oxazole CH).

b) 3-[2-(4-Methyl-1,3-oxazol)-yl]-1,2,5,6-tetrahydropyridine hydrogen oxalate

A solution of the foregoing carbamate (1.03g, 4.40mmol) in methanolic hydrogen chloride (30ml) was stirred at 20° for 15h, and evaporated to dryness. The residue was partitioned between aqueous potassium carbonate and dichloromethane, the organic layer dried over magnesium sulphate and evaporated to yield the title compound free base (683mg, 95%). This material was treated with oxalic acid (360mg, 4.0 mmol) and recrystallised from methanol to give the hydrogen oxalate salt (725mg, 65% yield). m.p. 176-178°. (Found: C, 57.89%; H, 5.54%; N, 10.95%; $C_9H_{12}N_2O$. $C_2H_2O_4$ requires: C, 51.97%; H, 5.55%; N, 11.02%). m/e 164 ($M^+$); δ (250MHz, $D_2O$) 2.14 (3H, s, $CH_3$); 2.61-2.72 (2H, m, $5CH_2$); 3.43 (2H, t, J = 6Hz, $6CH_2$); 4.08 (2H, d, J = 2Hz, $2CH_2$); 6.92-6.98 (1H, m, 4CH); 7.56 (1H, s, oxazole CH).

EXAMPLE 24

3-[5-(2-Methyl-1,3-oxazol)-yl]-1,2,5,6-tetrahydropyridine hydrogen oxalate

a) 1-Ethenyloxycarbonyl-3-[5-(2-methyl-1,3-oxazol)-yl]-1,2,5,6-tetrahydropyridine

A stirred solution of 1-methyl-3-[5-(2-methyl-1,3-oxazol)-yl]-1,2,5,6-tetrahydropyridine (2.30g, 12.9mmol), from Example 22, in 1,2-dichloroethane (18ml) at -5° was treated with vinyl chloroformate (1.55ml, 16.8mmol), causing a precipitate to form. After 15 min the mixture was heated to reflux for 4h, cooled, diluted with dichloromethane (50ml) and washed in turn with 0.5M hydrochloric acid and saturated sodium hydrogen carbonate. The organic layer was dried over magnesium sulphate and evaporated to give the crude product (570mg) which was purified by column chromatography on silica in dichloromethane/ethyl acetate (95:5). The third-eluted component was the desired product (22mg), as a gum. δ (250MHz, $CDCl_3$) 2.32-2.41 (2H, m, $5CH_2$); 2.47 (3H, s, $CH_3$); 3.60-3.71 (2H, m, $6CH_2$); 4.19-4.26 (2H, m, $2CH_2$); 4.50 (1H, d, J = 6Hz, $CO_2CH=CH$, trans to O); 4.84 (1H, d, J = 14Hz, $CO_2CH=CH$, cis to O); 6.29-6.38 (1H, m, 4CH); 6.83 (1H, s, oxazole CH); 7.25 (1H, dd, J = 6Hz and 14Hz, $CO_2CH=CH_2$).

b) 3-[5-(2-Methyl-1,3-oxazol)-yl]-1,2,5,6-tetrahydropyridine hydrogen oxalate

A solution of the foregoing carbamate (20mg, 0.086mmol) in methanolic hydrogen chloride was stirred at 20° for 3h, then partitioned between aqueous potassium carbonate and dichloromethane. The organic layer was dried over potassium carbonate and evaporated to yield the title compound (12mg, 85%), which was converted to the hydrogen oxalate and crystallised from ethanol/ether, m.p. 110-122°. m/e 164 ($M^+$); δ (250MHz, $D_2O$) 2.54 (3H, s, $CH_3$); 2.54-2.67 (2H, m, $5CH_2$); 3.41 (2H, t, J = 6Hz, $6CH_2$); 3.95-4.02 (2H, m, $2CH_2$); 6.52-6.60 (1H, m, 4CH); 7.11 (1H, s, oxazole CH).

EXAMPLE 25

Tablet Preparation

Tablets containing 1.0, 2.0, 25.0, 26.0, 50.0 and 100.0 mg, respectively, of the following compounds are prepared as illustrated below:

3-[4-(1,3-Thiazol)-yl]quinuclidine hydrogen oxalate
3-[2-(4-Methyl-1,3-oxazol)-yl]quinuclidine hydrochloride
3-[2-(5-methyl-1,3-oxazol)-yl]-1,2,5,6-tetrahydropyridine hydrochloride
3-[4-(2-Methyl-1,3-thiazol)-yl]-1,2,5,6-tetrahydropyridine dihydrochloride

TABLE FOR DOSES CONTAINING FROM

1-25 MG OF THE ACTIVE COMPOUND

| | Amount-mg | | |
|---|---|---|---|
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

TABLE FOR DOSES CONTAINING FROM

26-100 MG OF THE ACTIVE COMPOUND

| | Amount-mg | | |
|---|---|---|---|
| Active Compound | 26.0 | 50.0 | 100.0 |
| Microcrystalline Cellulose | 52.0 | 100.0 | 200.0 |
| Modified food corn starch | 2.21 | 4.25 | 8.5 |
| Magnesium stearate | 0.39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.00 mg, 50.0 mg and 100.0 mg of active ingredient per tablet.

**Claims**

**Claims for the following Contracting States : AT, BE, CH, DE, FR, GB, IT, LI, LU, NL, SE**

**1.** The use of a 1,3-oxazole or 1,3-thiazole of structural formula I, or a pharmaceutically acceptable salt or prodrug thereof:

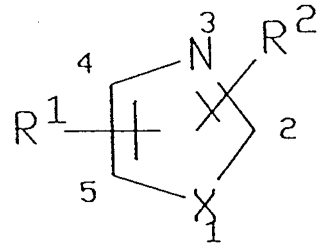

(I)

wherein

X represents oxygen or sulphur;

$R^1$ represents a non-aromatic azacyclic or azabicyclic ring system which system may be fused, spiro or bridged, containing one nitrogen atom as the sole heteroatom and from 4 to 10 ring atoms; and

$R^2$ represents hydrogen, halogen, $-CF_3$, $-OR^7$, $-NR^7R^8$, $-NHOR^7$, $-NHNH_2$, -CN, $-CO_2R^7$ or $-CONR^7R^8$, or a substituted or unsubstituted, saturated or unsaturated hydrocarbon group selected from $C_{1-15}$ alkyl, $C_{2-15}$ alkenyl, $C_{2-15}$ alkynyl, aryl and aralkyl; wherein $R^7$ and $R^8$ independently represent hydrogen or $C_{1-2}$ alkyl; provided that:

(a) when $R^2$ is hydrogen and $R^1$ is in the 2-position, then $R^1$ is in the endo configuration; and

(b) when $R^2$ is methyl and $R^1$ is in the 2-position, then $R^1$ does not represent an unsubstituted exo-1-azabicyclo[2.2.1]hept-3-yl, exo-1-azabicyclo[3.2.1]oct-3-yl or exo-1-azabicyclo[3.2.1]oct-6-yl group;

(c) when $R^1$ is 1,2,5,6-tetrahydropyrid-3-yl or piperid-3-yl, optionally substituted on a ring carbon or nitrogen atom thereof by $C_{1-4}$ alkyl, then $R^2$ is other than hydrogen, $-CF_3$, $-OR^7$ or $C_{1-6}$ alkyl optionally substituted by halogen, -OH or phenyl which may itself be optionally substituted by halo, $C_{1-6}$ alkoxy, $C_{1-6}$ alkyl or $-CF_3$;

for the preparation of a medicament useful for the treatment of neurological and mental illnesses whose clinical manifestations are due to involvement of cholinergic neurones.

**2.** The use of a compound of formula I as defined in claim 1, or a pharmaceutically acceptable salt or prodrug thereof, for the preparation of a medicament useful for the treatment of severe painful conditions.

**3.** A compound of formula II, or a salt or prodrug thereof:

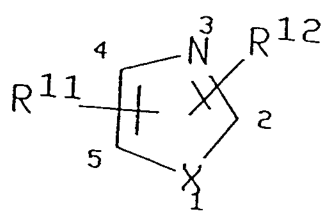

(II)

wherein

X is oxygen or sulphur;

$R^{11}$ represents a non-aromatic azacyclic or azabicyclic ring system which system may be fused, spiro or bridged, containing one nitrogen atom as the sole heteroatom and from 4 to 10 ring atoms; and

$R^{12}$ represents hydrogen, halogen, $-CF_3$, $-OR^7$, $-NR^7R^8$, $-NHOR^7$, $-NHNH_2$, -CN, $-CO_2R^7$ or $-CONR^7R^8$ or an optionally substituted, saturated or unsaturated hydrocarbon group selected from $C_{1-15}$ alkyl, $C_{2-15}$ alkenyl, $C_{2-15}$ alkynyl, aryl and aralkyl; wherein $R^7$ and $R^8$ independently represent

hydrogen or $C_{1-2}$ alkyl;

provided that when $R^{12}$ is present in the 2-position and represents amino, alkylamino, dialkylamino or alkylcarbonylamino in which the alkyl moieties have from 1-4 carbon atoms, then $R^{11}$, when present in the 4-position, does not represent optionally N-substituted piperidin-3-yl or 1,2,5,6-tetrahydropyridin-3-yl;

provided also that:

(a) when $R^{12}$ is hydrogen and $R^{11}$ is in the 2-position, then $R^{11}$ is in the endo configuration; and

(b) when $R^{12}$ is methyl and $R^{11}$ is in the 2-position, then $R^{11}$ does not represent an unsubstituted exo-1-azabicyclo[2.2.1]hept-3-yl, exo-1-azabicyclo[3.2.1]oct-3-yl or exo-1-azabicyclo[3.2.1]oct-6-yl group; and

(c) when $R^1$ is 1,2,5,6-tetrahydropyrid-3-yl or piperid-3-yl, optionally substituted on a ring carbon or nitrogen atom thereof by $C_{1-4}$ alkyl, then $R^2$ is other than hydrogen, $-CF_3$, $-OR^7$ or $C_{1-6}$ alkyl optionally substituted by halogen, -OH or phenyl which may itself be optionally substituted by halo, $C_{1-6}$ alkoxy, $C_{1-6}$ alkyl or $-CF_3$.

**4.** A compound as claimed in claim 3 represented by formula III:

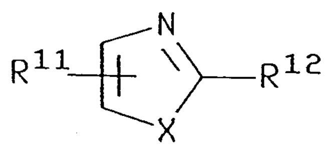

(III)

wherein X, $R^{11}$ and $R^{12}$ are as defined in claim 3.

**5.** A compound as claimed in claim 3 represented by the formula IV:

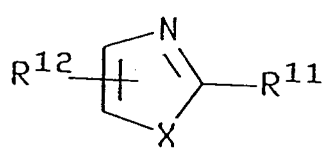

(IV)

wherein X, $R^{11}$ and $R^{12}$ are as defined in claim 3.

**6.** A compound as claimed in any one of claims 3 to 5 wherein $R^{11}$ represents tetrahydropyridine, 1-azanorbornane, guinuclidine or 1-azabicyclo[3.2.1]octane, any of which groups $R^{11}$ may be substituted with $C_{1-3}$ alkyl or hydroxy.

**7.** A compound as claimed in any one of claims 3 to 6 wherein $R^{12}$ represents hydrogen, methyl, ethyl, amino or hydroxy.

**8.** A compound selected from:

3-[4-(2-amino-1,3-thiazol)-yl]quinuclidine;
3-[4-(1,3-thiazol)-yl]quinuclidine;
3-[4-(2-amino-1,3-thiazol)-yl]pyrrolidine;

3-[4-(2-amino-1,3-thiazol)-yl]-1-methylpyrrolidine;
3-[4-(2-methyl-1,3-thiazol)-yl]quinuclidine;
3-[4-(2-hydroxy-1,3-thiazol)-yl]quinuclidine;
3-[5-(2-methyl-1,3-thiazol)-yl]quinuclidine;
3-[4-(2-amino-1,3-thiazol)-yl]-1-azabicyclo[2.2.1]heptane;
3-[5-(2-methyl-1,3-oxazol)-yl]quinuclidine;
3-[5-(2-methyl-1,3-oxazol)-yl]-1-azabicyclo[2.2.1]heptane;
3-[4-(2-methyl-1,3-oxazol)-yl]quinuclidine;
3-[4-(2-amino-1,3-oxazol)-yl]quinuclidine;
3-[2-(4-methyl-1,3-thiazol)-yl]quinuclidine;
3-[2-(4-methyl-1,3-oxazol)-yl]quinuclidine;
3-[2-(5-methyl-1,3-oxazol)-yl]quinuclidine;
3-[2-(4-methyl-1,3-thiazol)-yl]-1-azabicyclo[2.2.2]oct-2-ene;
3-hydroxy-3-[2-(4-methyl-1,3-oxazol)-yl]quinuclidine;
(+)-3-[2-(4-methyl-1,3-oxazol)-yl]quinuclidine;
(-)-3-[2-(4-methyl-1,3-oxazol)-yl]quinuclidine;
3-hydroxy-3-[2-(1,3-oxazol)-yl]quinuclidine;
3-hydroxy-3-[2-(4-ethyl-1,3-oxazol)-yl]quinuclidine;
3-[2-(4-ethyl-1,3-oxazol)-yl]quinuclidine;
3-hydroxy-3-[2-(4-methyl-1,3-thiazol)-yl]quinuclidine;
1-methyl-3-[2-(5-methyl-1,3-oxazol)-yl]piperidine;
1-methyl-3-[2-(5-methyl-1,3-oxazol)-yl]-1,2,5,6-tetrahydropyridine;
3-[2-(5-methyl-1,3-oxazol)-yl]-1,2,5,6-tetrahydropyridine;
1-methyl-3-[4-(2-methyl-1,3-thiazol)-yl]-1,2,5,6-tetrahydropyridine;
3-[4-(2-methyl-1,3-thiazol)-yl]-1,2,5,6-tetrahydropyridine;
1-methyl-3-[4-(2-methyl-1,3-oxazol)-yl]-1,2,5,6-tetrahydropyridine;
3-[4-(2-methyl-1,3-oxazol)-yl]-1,2,5,6-tetrahydropyridine;
1-methyl-3-[2-(4-methyl-1,3-oxazol)-yl]-1,2,5,6-tetrahydropyridine;
1-methyl-3-[5-(2-methyl-1,3-oxazol)-yl]-1,2,5,6-tetrahydropyridine;
3-[2-(4-methyl-1,3-oxazol)-yl]-1,2,5,6-tetrahydropyridine;
3-[5-(2-methyl-1,3-oxazol)-yl]-1,2,5,6-tetrahydropyridine;

and salts and prodrugs thereof.

**9.** A pharmaceutical composition comprising a compound as claimed in any one of claims 3 to 8 in association with a pharmaceutically acceptable carrier.

**10.** A pharmaceutical composition as claimed in claim 9 further comprising a peripheral cholinergic antagonist.

**11.** A process for the preparation of a compound as claimed in any one of claims 3 to 8, which process comprises:

(A) reacting a compound of formula $R^aCO.CH_2Y$, wherein Y represents bromo, chloro or iodo; with a compound of formula $R^bCX.NH_2$, wherein X is as defined in claim 3, and one of $R^a$ and $R^b$ represents a group of formula $R^{11}$ as defined in claim 3 and the other represents a group of formula $R^{12}$ as defined in claim 3; or

(B) dehydroxylating a compound of formula V:

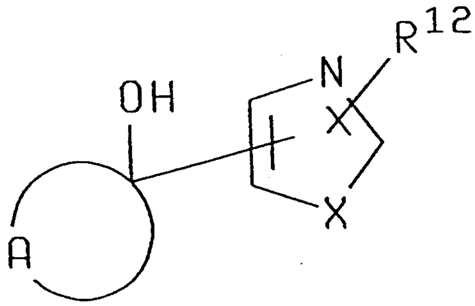

(V)

wherein $R^{12}$ and X are as defined in claim 3, and A represents the residue of an azacyclic or azabicyclic ring system which system may be fused, spiro or bridged, containing one nitrogen atom as the sole heteroatom and from 4 to 10 ring atoms; or

(C) for the preparation of a compound of formula II wherein X represents sulphur:
cyclisation of an acylaminoketone of formula $R^aCOCH_2NHCOR^b$, wherein $R^a$ and $R^b$ are as defined above, in the presence of a dehydrating sulphide; or

(D) for the preparation of a compound of formula II wherein X represents oxygen:
cyclisation of an acylaminoketone of formula $R^aCOCH_2NHCOR^b$, wherein $R^a$ and $R^b$ are as defined above, in the presence of a dehydrating agent; or

(E) for the preparation of a compound of formula II wherein X represents oxygen:
cyclisation of a ketal of formula $R^aCOCH_2NHCR^bR^fR^g$ in which $R^a$ and $R^b$ are as defined above and $R^f$ and $R^g$ independently represent $C_{1-4}$ alkoxy; or

(f) for the preparation of a compound of formula II wherein X represents oxygen:
reacting an aldehyde of formula $R^aCHO$ with the anion of an isonitrile of formula $L\text{-}CH_2N=C(Z)R^b$, wherein L represents a leaving group, Z represents methoxy or methylthio, and $R^a$ and $R^b$ are as defined above; or

(G) for the preparation of a compound of formula II wherein X represents oxygen and $R^{12}$ represents an optionally monosubstituted methyl group in the 5-position:
cyclisation of a propargyl amide of formula XI:

$R^{11}CONHCH_2C{\equiv}CR^x$     (XI)

wherein $R^{11}$ is as defined in claim 3 and $R^x$ is a group such that $-CH_2R^x$ represents the group $R^{12}$ as defined in claim 3, by treatment with mercuric acetate in acetic acid; or

(H) for the preparation of a compound of formula II wherein X represents oxygen and $R^{12}$ represents an optionally monosubstituted methyl group in the 4-position:
reacting a cyano compound of formula $R^{11}CN$ with a propargyl alcohol of formula $HOCH_2C{\equiv}CR^x$, wherein $R^{11}$ is as defined in claim 3 and $R^x$ is as defined above.

**Claims for the following Contracting State : ES**

1. The use of a 1,3-oxazole or 1,3-thiazole of structural formula I, or a pharmaceutically acceptable salt or prodrug thereof:

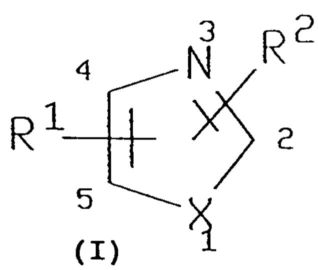

(I)

wherein

X represents oxygen or sulphur;

$R^1$ represents a non-aromatic azacyclic or azabicyclic ring system which system may be fused, spiro or bridged, containing one nitrogen atom as the sole heteroatom and from 4 to 10 ring atoms; and

$R^2$ represents hydrogen, halogen, $-CF_3$, $-OR^7$, $-NR^7R^8$, $-NHOR^7$, $-NHNH_2$, -CN, $-CO_2R^7$ or $-CONR^7R^8$, or a substituted or unsubstituted, saturated or unsaturated hydrocarbon group selected from $C_{1-15}$ alkyl, $C_{2-15}$ alkenyl, $C_{2-15}$ alkynyl, aryl and aralkyl, wherein $R^7$ and $R^8$ independently represent hydrogen or $C_{1-2}$ alkyl; provided that:

(a) when $R^2$ is hydrogen and $R^1$ is in the 2-position, then $R^1$ is in the endo configuration; and

(b) when $R^2$ is methyl and $R^1$ is in the 2-position, then $R^1$ does not represent an unsubstituted exo-1-azabicyclo[2.2.1]hept-3-yl, exo-1-azabicyclo[3.2.1]oct-3-yl or exo-1-azabicyclo[3.2.1]oct-6-yl group;

(c) when $R^1$ is 1,2,5,6-tetrahydropyrid-3-yl or piperid-3-yl, optionally substituted on a ring carbon or nitrogen atom thereof by $C_{1-4}$ alkyl, then $R^2$ is other than hydrogen, $-CF_3$, $-OR^7$ or $C_{1-6}$ alkyl optionally substituted by halogen, -OH or phenyl which may itself be optionally substituted by halo, $C_{1-6}$ alkoxy, $C_{1-6}$ alkyl or $-CF_3$

for the preparation of a medicament useful for the treatment of neurological and mental illnesses whose clinical manifestations are due to involvement of cholinergic neurones.

**2.** The use of a compound of formula I as defined in claim 1, or a pharmaceutically acceptable salt or prodrug thereof, for the preparation of a medicament useful for the treatment of severe painful conditions.

**3.** A process for the preparation of a compound of formula II, or a salt or prodrug thereof:

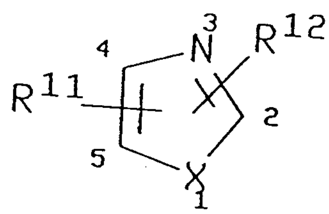

(II)

wherein

X is oxygen or sulphur;

$R^{11}$ represents a non-aromatic azacyclic or azabicyclic ring system which system may be fused, spiro or bridged, containing one nitrogen atom as the sole heteroatom and from 4 to 10 ring atoms; and

$R^{12}$ represents hydrogen, halogen, $-CF_3$, $-OR^7$, $-NR^7R^8$, $-NHOR^7$, $-NHNH_2$, -CN, $-CO_2R^7$ or $-CONR^7R^8$ or an optionally substituted, saturated or unsaturated hydrocarbon group selected from $C_{1-15}$ alkyl, $C_{2-15}$ alkenyl, $C_{2-15}$ alkynyl, aryl and aralkyl, wherein $R^7$ and $R^8$ independently represent hydrogen or $C_{1-2}$ alkyl;

provided that when $R^{12}$ is present in the 2-position and represents amino, alkylamino, dialkylamino or alkylcarbonylamino, in which the alkyl moieties have from 1-4 carbon atoms, then $R^{11}$, when present in the 4-position, does not represent optionally N-substituted piperidin-3-yl or 1,2,5,6-tetrahydropyridin-3-yl;

provided also that:

(a) when $R^{12}$ is hydrogen and $R^{11}$ is in the 2-position, then $R^{11}$ is in the endo configuration; and

(b) when $R^{12}$ is methyl and $R^{11}$ is in the 2-position, then $R^{11}$ does not represent an unsubstituted exo-1-azabicyclo[2.2.1]hept-3-yl, exo-1-azabicyclo[3.2.1]oct-3-yl or exo-1-azabicyclo[3.2.1]oct-6-yl group;

(c) when $R^1$ is 1,2,5,6-tetrahydropyrid-3-yl or piperid-3-yl, optionally substituted on a ring carbon or nitrogen atom thereof by $C_{1-4}$ alkyl, then $R^2$ is other than hydrogen, $-CF_3$, $-OR^7$ or $C_{1-6}$ alkyl optionally substituted by halogen, -OH or phenyl which may itself be optionally substituted by halo, $C_{1-6}$ alkoxy, $C_{1-6}$ alkyl or $-CF_3$ which process comprises:

(A) reacting a compound of formula $R^aCO.CH_2Y$, wherein Y represents bromo, chloro or iodo; with a

compound of formula $R^bCX.NH_2$, wherein X is as defined above, and one of $R^a$ and $R^b$ represents a group of formula $R^{11}$ as defined above and the other represents a group of formula $R^{12}$ as defined above; or

(B) dehydroxylating a compound of formula V:

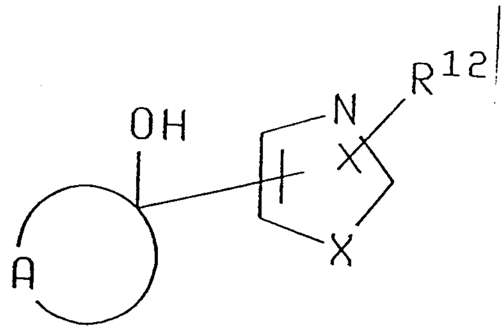

(V)

wherein $R^{12}$ and X are as defined above, and A represents the residue of an azacyclic or azabicyclic ring system which system may be fused, spiro or bridged, containing one nitrogen atom as the sole heteroatom and from 4 to 10 ring atoms; or

(C) for the preparation of a compound of formula II wherein X represents sulphur:
cyclisation of an acylaminoketone of formula $R^aCOCH_2NHCOR^b$, wherein $R^a$ and $R^b$ are as defined above, in the presence of a dehydrating sulphide; or

(D) for the preparation of a compound of formula II wherein X represents oxygen:
cyclisation of an acylaminoketone of formula $R^aCOCH_2NHCOR^b$, wherein $R^a$ and $R^b$ are as defined above, in the presence of a dehydrating agent; or

(E) for the preparation of a compound of formula II wherein X represents oxygen:
cyclisation of a ketal of formula $R^aCOCH_2NHCR^bR^fR^g$ in which $R^a$ and $R^b$ are as defined above and $R^f$ and $R^g$ independently represent $C_{1-4}$ alkoxy; or

(F) for the preparation of a compound of formula II wherein X represents oxygen:
reacting an aldehyde of formula $R^aCHO$ with the anion of an isonitrile of formula $L\text{-}CH_2N=C(Z)R^b$, wherein L represents a leaving group, Z represents methoxy or methylthio, and $R^a$ and $R^b$ are as defined above; or

(G) for the preparation of a compound of formula II wherein X represents oxygen and $R^{12}$ represents an optionally monosubstituted methyl group in the 5-position:
cyclisation of a propargyl amide of formula XI:

$R^{11}CONHCH_2C=CR^x$ (XI)

wherein $R^{11}$ is as defined above and $R^x$ is a group such that $-CH_2R^x$ represents the group $R^{12}$ as defined above, by treatment with mercuric acetate in acetic acid; or

(H) for the preparation of a compound of formula II wherein X represents oxygen and $R^{12}$ represents an optionally monosubstituted methyl group in the 4-position:
reacting a cyano compound of formula $R^{11}CN$ with a propargyl alcohol of formula $HOCH_2C{\equiv}CR^x$, wherein $R^{11}$ and $R^x$ are as defined above.

**4.** A process as claimed in claim 3 for the preparation of a compound represented by formula III:

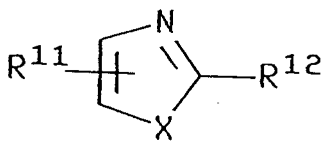

(III)

wherein X, $R^{11}$ and $R^{12}$ are as defined in claim 3.

**5.** A process as claimed in claim 3 for the preparation of a compound represented by the formula IV:

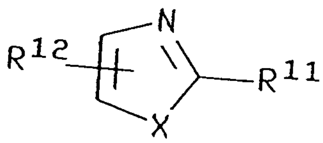

(IV)

wherein X, $R^{11}$ and $R^{12}$ are as defined in claim 3.

**6.** A process as claimed in any one of claims 3 to 5 for the preparation of a compound wherein $R^{11}$ represents tetrahydropyridine, 1-azanorbornane, quinuclidine or 1-azabicyclo[3.2.1]octane, any of which groups $R^{11}$ may be substituted with $C_{1-3}$ alkyl or hydroxy.

**7.** A process as claimed in any one of claims 3 to 6 for the preparation of a compound wherein $R^{12}$ represents a hydrogen, methyl, ethyl, amino or hydroxy.

**8.** A process as claimed in any one of claims 3 to 7 for the preparation of a compound selected from:

3-[4-(2-amino-1,3-thiazol)-yl]quinuclidine;
3-[4-(1,3-thiazol)-yl]quinuclidine;
3-[4-(2-amino-1,3-thiazol)-yl]pyrrolidine;
3-[4-(2-amino-1,3-thiazol)-yl]-1-methylpyrrolidine;
3-[4-(2-methyl-1,3-thiazol)-yl]quinuclidine;
3-[4-(2-hydroxy-1,3-thiazol)-yl]quinuclidine;
3-[5-(2-methyl-1,3-thiazol)-yl]quinuclidine;
3-[4-(2-amino-1,3-thiazol)-yl]-1-azabicyclo[2.2.1]heptane;
3-[5-(2-methyl-1,3-oxazol)-yl]quinuclidine:
3-[5-(2-methyl-1,3-oxazol)-yl]-1-azabicyclo[2.2.1]heptane;
3-[4-(2-methyl-1,3-oxazol)-yl]quinuclidine;
3-[4-(2-amino-1,3-oxazol)-yl]quinuclidine;
3-[2-(4-methyl-1,3-thiazol)-yl]quinuclidine;
3-[2-(4-methyl-1,3-oxazol)-yl]quinuclidine;
3-[2-(5-methyl-1,3-oxazol)-yl]quinuclidine;
3-[2-(4-methyl-1,3-thiazol)-yl]-1-azabicyclo[2.2.2]oct-2-ene;
3-hydroxy-3-[2-(4-methyl-1,3-oxazol)-yl]quinuclidine;
(+)-3-[2-(4-methyl-1,3-oxazol)-yl]quinuclidine;
(-)-3-[2-(4-methyl-1,3-oxazol)-yl]quinuclidine;
3-hydroxy-3-[2-(1,3-oxazol)-yl]quinuclidine;
3-hydroxy-3-[2-(4-ethyl-1,3-oxazol)-yl]quinuclidine;
3-[2-(4-ethyl-1,3-oxazol)-yl]quinuclidine;
3-hydroxy-3-[2-(4-methyl-1,3-thiazol)-yl]quinuclidine;
1-methyl-3-[2-(5-methyl-1,3-oxazol)-yl]piperidine;
1-methyl-3-[2-(5-methyl-1,3-oxazol)-yl]-1,2,5,6-tetrahydropyridine;

3-[2-(5-methyl-1,3-oxazol)-yl]-1,2,5,6- tetrahydropyridine;
1-methyl-3-[4-(2-methyl-1,3-thiazol)-yl]-1,2,5,6-tetrahydropyridine;
3-[4-(2-methyl-1,3-thiazol)-yl]-1,2,5,6-tetrahydropyridine;
1-methyl-3-[4-(2-methyl-1,3-oxazol)-yl]-1,2,5,6-tetrahydropyridine;
3-[4-(2-methyl-1,3-oxazol)-yl]-1,2,5,6-tetrahydropyridine;
1-methyl-3-[2-(4-methyl-1,3-oxazol)-yl]-1,2,5,6-tetrahydropyridine;
1-methyl-3-[5-(2-methyl-1,3-oxazol)-yl]-1,2,5,6-tetrahydropyridine;
3-[2-(4-methyl-1,3-oxazol)-yl]-1,2,5,6-tetrahydropyridine;
3-[5-(2-methyl-1,3-oxazol)-yl]-1,2,5,6-tetrahydropyridine;

and salts and prodrugs thereof.

**9.** A process for the preparation of a pharmaceutical composition which comprises mixing a compound prepared as claimed in any one of claims 3 to 8 with a pharmaceutically acceptable carrier.

**10.** A process as claimed in claim 9 which further comprises incorporating a peripheral cholinergic antagonist into the composition.

**Patentansprüche**

**Patentansprüche für folgende Vertragsstaaten : AT, BE, CH, DE, FR, GB, IT, LI, LU, NL, SE**

**1.** Verwendung eines 1,3-Oxazols oder 1,3-Thiazols der Strukturformel I oder eines pharmazeutisch annehmbaren Salzes oder Pro-Pharmakons davon:

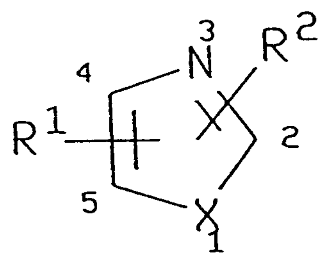

(I)

wobei

X Sauerstoff oder Schwefel bedeutet,

$R^1$ ein nicht aromatisches azacyclisches oder azabicyclisches Ringsystem bedeutet, das kondensiert, spiro- oder durch eine Brücke verbunden sein kann, und ein Stickstoffatom als einziges Heteroatom und 4 bis 10 Ringatome enthält, und

$R^2$ Wasserstoff, Halogen, $-CF_3$, $-OR^7$, $-NR^7R^8$, $-NHOR^7$, $-NHNH_2$, -CN, $-CO_2R^7$ oder $-COHR^7R^8$ oder eine substituierte oder unsubstituierte gesättigte oder ungesättigte Kohlenwasserstoffgruppe, ausgewählt aus $C_1$-$C_{15}$-Alkyl, $C_2$-$C_{15}$-Alkenyl, $C_2$-$C_{15}$-Alkinyl, Aryl und Aralkyl, bedeutet, wobei $R^7$ und $R^8$ unabhängig voneinander Wasserstoff oder $C_1$-$C_2$-Alkyl bedeuten, mit der Maßgabe, daß:

(a) wenn $R^2$ Wasserstoff bedeutet und $R^1$ in 2-Stellung angeordnet ist, sich $R^1$ in der Endo-Konfiguration befindet, und

(b) wenn $R^2$ Methyl bedeutet und $R^1$ sich in 2-Stellung befindet, $R^1$ keine unsubstituierte exo-1-Azabicyclo[2,2,1]hept-3-yl, exo-1-Azabicyclo[3,2,1]oct-3-yl oder exo-1-Azabicyclo-[3,2,1]oct-6-yl-Gruppe ist;

(c) wenn $R^1$ gegebenenfalls an einem Ringkohlenstoffatom oder Stickstoffatom durch $C_1$-$C_4$-Alkyl substituiertes 1,2,5,6-Tetrahyrdopyrid-3-yl oder Piperid-3-yl bedeutet, dann $R^2$ kein Wasserstoff, $-CF_3$, $-OR^7$ oder $C_1$-$C_6$-Alkyl, gegebenenfalls substituiert durch Halogen, -OH oder Phenyl, das wiederum gegebenenfalls durch Halogen, $C_1$-$C_6$-Alkoxy, $C_1$-$C_6$-Alkyl oder $-CF_3$ substituiert sein kann, bedeutet;

zur Herstellung eines Arzneimittels, das geeignet ist zur Behandlung von neurologischen und mentalen Erkrankungen, deren klinische Manifestationen auf einer Beteiligung cholinerger Meuronen beruhen.

**2.** Verwendung einer Verbindung der Formel I, wie in Anspruch 1 definiert, oder eines pharmazeutisch

annehmbaren Salzes oder Pro-Pharmakons davon, zur Herstellung eines Arzneimittels, das geeignet ist zur Behandlung von schweren Schmerzzuständen.

3. Verbindung der Formel II oder ein Salz oder Pro-Pharmakon davon:

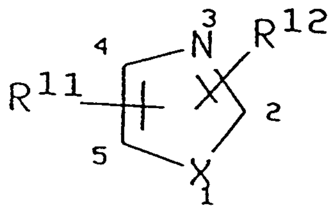

(II)

wobei

X Sauerstoff oder Schwefel bedeutet,

$R^{11}$ ein nicht aromatisches azacyclisches oder azabicyclisches Ringsystem bedeutet, das kondensiert, spiro- oder durch eine Brücke verbunden sein kann, und ein Stickstoffatom als einziges Heteroatom und 4 bis 10 Ringatome enthält, und

$R^{12}$ Wasserstoff, Halogen, $-CF_3$, $-OR^7$, $-NR^7R^8$, $-NHOR^7$, $-NHNH_2$, -CN, $-CO_2R^7$ oder $-CONR^7R^8$ oder eine ggf.substituierte oder unsubstituierte gesättigte oder ungesättigte Kohlenwasserstoffgruppe, ausgewählt aus $C_1$-$C_{15}$-Alkyl, $C_2$-$C_{15}$-Alkenyl, $C_2$-$C_{15}$-Alkinyl, Aryl und Aralkyl bedeutet, wobei $R^7$ und $R^8$ unabhängig voneinander Wasserstoff oder $C_1$-$C_2$-Alkyl bedeuten,mit der Maßgabe, daß

wenn $R^{12}$ in 2-Stellung vorhanden ist und Amino, Alkylamino, Dialkylamino oder Alkylcarbonylamino bedeutet, wobei die Alkyleinheiten 1 bis 4 Kohlenstoffatome aufweist, $R^{11}$, soweit in 4-Stellung vorhanden, kein gegebenenfalls N-substituiertes Piperidin-3-yl oder 1,2,5,6-Tetrahydropyridin-3-yl bedeutet;

mit der weiteren Maßgabe, daß:

(a) wenn $R^{12}$ Wasserstoff bedeutet und $R^{11}$ in 2-Stellung angeordnet ist, sich $R^{11}$ in der Endo-Konfiguration befindet, und

(b) wenn $R^{12}$ Methyl bedeutet und $R^{11}$ sich in 2-Stellung befindet, $R^{11}$ keine unsubstituierte exo-1-Azabicyclo[2,2,1]hept-3-yl-,exo-1-Azabicyclo[3,2,1]oct-3-yl- oder exo-1-Azabicyclo[3,2,1]oct-6-yl-Gruppe ist;

(c) wenn $R^1$ gegebenenfalls an einem Ringkohlenstoffatom oder Stickstoffatom durch $C_1$-$C_4$-Alkyl substituiertes 1,2,5,6-Tetrahydropyrid-3-yl oder Piperid-3-yl bedeutet, dann $R^1$ kein Wasserstoff, $-CF_3$, $-OR^7$ oder $C_1$-$C_6$-Alkyl, gegebenenfalls substituiert durch Halogen, -OH oder Phenyl, das wiederum gegebenenfalls durch Halogen, $C_1$-$C_6$-Alkoxy, $C_1$-$C_6$-Alkyl oder $-CF_3$ substituiert sein kann, bedeutet.

4. Verbindung nach Anspruch 3, angegeben durch die Formel III:

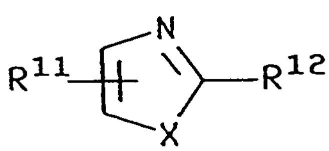

(III)

in der X, $R^{11}$ und $R^{12}$ wie in Anspruch 3 definiert sind.

5. Verbindung nach Anspruch 3, angegeben durch die Formel IV:

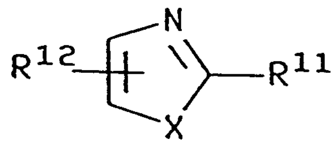

(IV)

wobei X, $R^{11}$ und $R^{12}$ wie in Anspruch 3 definiert sind.

6. Verbindung nach einem der Ansprüche 3 bis 5, wobei $R^{11}$ Tetrahydropyridin, 1-Azanorbornan, Chinuclidin oder 1-Azabicyclo[3,2,1]octan bedeutet, wobei jede der Gruppen $R^{11}$ durch $C_1$-$C_3$-Alkyl oder Hydroxy substituiert sein kann.

7. Verbindung nach einem der Ansprüche 3 bis 6, wobei $R^{12}$ Wasserstoff, Methyl, Ethyl, Amino oder Hydroxy bedeutet.

8. Verbindung, ausgewählt aus:

3-[4-(2-Amino-1,3-thiazol)-yl]chinuclidin;
3-[4-(1,3-Thiazol)-yl]chinuclidin;
3-[4-(2-Amino-1,3-thiazol)-yl]pyrrolidin;
3-[4-(2-Amino-1,3-thiazol)-yl]-1-methylpyrrolidon;
3-[4-(2-Methyl-1,3-thiazol)-yl]chinuclidin;
3-[4-(2-Hydroxy-1,3-thiazol)-yl]chinuclidin;
3-[5-(2-Methyl-1,3-thiazol)-yl]chinuclidin;
3-[4-(2-Amino-1,3-thiazol)-yl]-1-azabicyclo[2,2,1]heptan;
3-[5-(2-Methyl-1,3-oxazol)-yl]chinuclidin;
3-[5-(2-Methyl-1,3-oxazol)-yl]-1-azabicyclo[2,2,1]heptan;
3-[4-(2-Methyl-1,3-oxazol)-yl]chinuclidin;
3-[4-(2-Amino-1,3-oxazol)-yl]chinuclidin;
3-[2-(4-Methyl-1,3-thiazol)-yl]chinuclidin;
3-[2-(4-Methyl-1,3-oxazol)-yl]chinuclidin;
3-[2-(5-Methyl-1,3-oxazol)-yl]chinuclidin;
3-[2-(4-Methyl-1,3-thiazol)-yl]-1-azabicyclo[2,2,2]oct-2-en;
3-Hydroxy-3-[2-(4-methyl-1,3-oxazol)-yl]chinuclidin;
(+)-3-[2-(4-Methyl-1,3-oxazol)-yl]chinuclidin;
(-)-3-[2-(4-Methyl-1,3-oxazol)-yl]chinuclidin;
3-Hydroxy-3-[2-(1,3-oxazol)-yl]chinuclidin;
3-Hydroxy-3-[2-(4-ethyl-1,3-oxazol)-yl]chinuclidin;
3-[2-(4-Ethyl-1,3-oxazol)-yl]chinuclidin;
3-Hydroxy-3-[2-(4-methyl-1,3-thiazol)-yl]chinuclidin;
1-Methyl-3-[2-(5-methyl-1,3-oxazol)-yl]piperidin;
1-Methyl-3-[2-(5-methyl-1,3-oxazol)-yl]-1,2,5,6-tetrahydropyridin;
3-[2-(5-Methyl-1,3-oxazol)-yl]-1,2,5,6-tetrahydropyridin;
1-Methyl-3-[4-(2-methyl-1,3-thiazol)-yl]-1,2,5,6-tetrahydropyridin;
3-[4-(2-Methyl-1,3-thiazol)-yl]-1,2,5,6-tetrahydropyridin;
1-Methyl-3-[4-(2-methyl-1,3-oxazol)-yl]-1,2,5,6-tetrahydropyridin;
3-[4-(2-Methyl-1,3-oxazol)-yl]-1,2,5,6-tetrahydropyridin;
1-Methyl-3-[2-(4-methyl-1,3-oxazol)-yl]-1,2,5,6-tetrahydropyridin;
1-Methyl-3-[5-(2-methyl-1,3-oxazol)-yl]-1,2,5,6-tetrahydropyridin;
3-[2-(4-Methyl-1,3-oxazol)-yl]-1,2,5,6-tetrahydropyridin;
3-[5-(2-Methyl-1,3-oxazol)-yl]-1,2,5,6-tetrahydropyridin

und Salzen und Pro-Pharmaka davon.

9. Pharmazeutisches Mittel, umfassend eine Verbindung nach einem der Ansprüche 3 bis 8 zusammen mit einem pharmazeutisch annehmbaren Träger.

10. Pharmazeutisches Mittel nach Anspruch 9, ferner umfassend einen peripheren cholinergen Antagonisten.

**11.** Verfahren zur Herstellung einer Verbindung nach einem der Ansprüche 3 bis 8, wobei das Verfahren umfaßt:

(A) das Umsetzen einer Verbindung der Formel $R^aCO.CH_2Y$, wobei Y Brom, Chlor oder Jod bedeutet, mit einer Verbindung der Formel $R^bCX.NH_2$, wobei X wie in Anspruch 3 definiert ist, und einer der Reste $R^a$ und $R^b$ eine Gruppe der Formel $R^{11}$, wie in Anspruch 3 definiert, bedeutet und der andere eine Gruppe der Formel $R^{12}$, wie in Anspruch 3 definiert, bedeutet oder

(B) Dehydroxylieren einer Verbindung der Formel V:

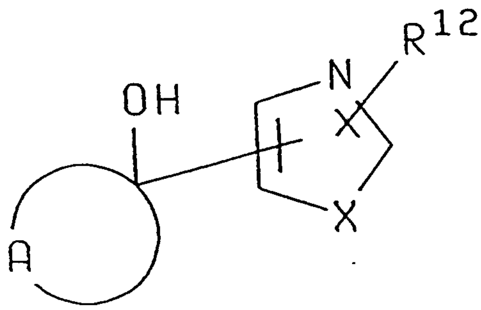

(V)

in der $R^{12}$ und X wie in Anspruch 3 definiert sind, A den Rest eines azacyclischen oder azabicyclischen Ringsystems bedeutet, wobei das Ringsystem kondensiert, spiro oder überbrückt sein kann, enthaltend ein Stickstoffatom als einziges Heteroatom und 4 bis 10 Ringatome; oder

(C) zur Herstellung einer Verbindung der Formel II, in der X Schwefel bedeutet:
Cyclisieren eines Acylaminoketons der Formel $R^aCOCH_2NHCOR^b$, wobei $R^a$ und $R^b$ wie oben definiert sind, in Gegenwart eines dehydratisierenden Sulfids oder

(D) zur Herstellung einer Verbindung der Formel II, in der X Sauerstoff bedeutet:
Cyclisieren eines Acylaminoketons der Formel $R^aCOCH_2NHCOR^b$, wobei $R^a$ und $R^b$ wie oben definiert sind, in Gegenwart eines Dehydratisierungsmittels oder

(E) zur Herstellung einer Verbindung der Formel II, in der X Sauerstoff bedeutet:
Cyclisieren eines Ketals der Formel $R^aCOCH_2NHCR^bR^fR^g$, in der $R^a$ und $R^b$ wie oben definiert sind und $R^f$ und $R^g$ unabhängig voneinander $C_1$-$C_4$-Alkoxy bedeuten, oder

(F) zur Herstellung einer Verbindung der Formel II, in der X Sauerstoff bedeutet:
Umsetzen eines Aldehyds der Formel $R^aCHO$ mit dem Anion eines Isonitrils der Formel $L\text{-}CH_2N=C\text{-}(Z)R^b$, in der L eine austretende Gruppe bedeutet, Z Methoxy oder Methylthio bedeutet und $R^a$ und $R^b$ wie oben definiert sind; oder

(G) zur Herstellung einer Verbindung der Formel II, in der X Sauerstoff bedeutet und $R^{12}$ eine gegebenenfalls einfach substituierte Methylgruppe in 5-Stellung ist:
Cyclisieren eines Propargylamids der Formel XI:

$$R^{11}CONHCH_2C{\equiv}CR^x \qquad (XI)$$

wobei $R^{11}$ wie in Anspruch 3 definiert ist und $R^x$ eine solche Gruppe bedeutet, daß $-CH_2R^x$ die Gruppe $R^{12}$, wie in Anspruch 3 definiert, bedeutet, durch Behandlung mit Quecksilberacetat in Essigsäure oder

(H) zur Herstellung einer Verbindung der Formel II, in der X Sauerstoff bedeutet und $R^{12}$ eine gegebenenfalls einfach substituierte Methylgruppe in 4-Stellung bedeutet:
Umsetzung einer Cyanoverbindung der Formel $R^{11}CN$ mit einem Propargylalkohol der Formel $HOCH_2C{\equiv}CR^x$, wobei $R^{11}$ wie in Anspruch 3 definiert ist und $R^x$ wie oben definiert ist.

**Patentansprüche für folgenden Vertragsstaat : ES**

**1.** Verwendung eines 1,3-Oxazols oder 1,3-Thiazols der Strukturformel I oder eines pharmazeutisch annehmbaren Salzes oder Pro-Pharmakons davon:

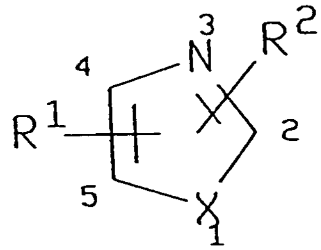

(I)

wobei

X Sauerstoff oder Schwefel bedeutet,

$R^1$ ein nicht aromatisches azacyclisches oder azabicyclisches Ringsystem bedeutet, das kondensiert, spiro- oder durch eine Brücke verbunden sein kann, und ein Stickstoffatom als einziges Heteroatom und 4 bis 10 Ringatome enthält, und

$R^2$ Wasserstoff, Halogen, $-CF_3$, $-OR^7$, $-NR^7R^8$, $-NHOR^7$, $-NHNH_2$, -CN, $-CO_2R^7$ oder $-CONR^7R^8$ oder eine substituierte oder unsubstituierte gesättigte oder ungesättigte Kohlenwasserstoffgruppe, ausgewählt aus $C_1$-$C_{15}$-Alkyl, $C_2$-$C_{15}$-Alkenyl, $C_2$-$C_{15}$-Alkinyl, Aryl und Aralkyl, bedeutet, wobei $R^7$ und $R^8$ unabhängig voneinander Wasserstoff oder $C_1$-$C_2$-Alkyl bedeuten, mit der Maßgabe, daß:

(a) wenn $R^2$ Wasserstoff bedeutet und $R^1$ in 2-Stellung angeordnet ist, sich $R^1$ in der Endo-Konfiguration befindet, und

(b) wenn $R^2$ Methyl bedeutet und $R^1$ sich in 2-Stellung befindet, $R^1$ keine unsubstituierte exo-1-Azabicyclo[2,2,1]hept-3-yl, exo-1-Azabicyclo[3,2,1]oct-3-yl oder exo-1-Azabicyclo-[3,2,1]oct-6-yl-Gruppe ist;

(c) wenn $R^1$ gegebenenfalls an einem Ringkohlenstoffatom oder Stickstoffatom durch $C_1$-$C_4$-Alkyl substituiertes 1,2,5,6-Tetrahyrdopyrid-3-yl oder Piperid-3-yl bedeutet, dann $R^2$ kein Wasserstoff, $-CF_3$, $-OR^7$ oder $C_1$-$C_6$-Alkyl, gegebenenfalls substituiert durch Halogen, -OH oder Phenyl, das wiederum gegebenenfalls durch Halogen, $C_1$-$C_6$-Alkoxy, $C_1$-$C_6$-Alkyl oder $-CF_3$ substituiert sein kann, bedeutet;

zur Herstellung eines Arzneimittels, das geeignet ist zur Behandlung von neurologischen und mentalen Erkrankungen, deren klinische Manifestationen auf einer Beteiligung cholinerger Neuronen beruhen.

**2.** Verwendung einer Verbindung der Formel I, wie in Anspruch 1 definiert, oder eines pharmazeutisch annehmbaren Salzes oder Pro-Pharmakons davon, zur Herstellung eines Arzneimittels, das geeignet ist zur Behandlung von schweren Schmerzzuständen.

**3.** Verfahren zur Herstellung einer Verbindung der Formel II oder eines Salzes oder Pro-Pharmakons davon

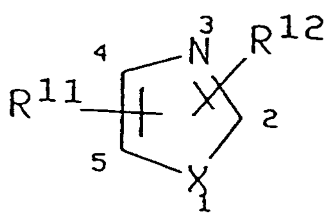

(II)

wobei

X Sauerstoff oder Schwefel bedeutet,

$R^{11}$ ein nicht aromatisches azacyclisches oder azabicyclisches Ringsystem bedeutet, das kondensiert, spiro- oder durch eine Brücke verbunden sein kann, und ein Stickstoffatom als einziges Heteroatom und 4 bis 10 Ringatome enthält, und

$R^{12}$ Wasserstoff, Halogen, $-CF_3$, $-OR^7$, $-NR^7R^8$, $-NHOR^7$, $-NHNH_2$, -CN, $-CO_2R^7$ oder $-CONR^7R^8$ oder eine ggf.substituierte oder unsubstituierte gesättigte oder ungesättigte Kohlenwasserstoffgruppe, ausgewählt aus $C_1$-$C_{15}$-Alkyl, $C_2$-$C_{15}$-Alkenyl, $C_2$-$C_{15}$-Alkinyl, Aryl und Aralkyl bedeutet, wobei $R^7$ und $R^8$ unabhängig voneinander Wasserstoff oder $C_1$-$C_2$-Alkyl bedeuten,

mit der Maßgabe, daß
wenn $R^{12}$ in 2-Stellung vorhanden ist und Amino, Alkylamino, Dialkylamino oder Alkylcarbonylamino bedeutet, wobei die Alkyleinheiten 1 bis 4 Kohlenstoffatome aufweist, $R^{11}$, soweit in 4-Stellung vorhanden, kein gegebenenfalls N-substituiertes Piperidin-3-yl oder 1,2,5,6-Tetrahydropyridin-3-yl bedeutet;
mit der weiteren Maßgabe, daß:
(a) wenn $R^{12}$ Wasserstoff bedeutet und $R^{11}$ in 2-Stellung angeordnet ist, sich $R^{11}$ in der Endo-Konfiguration befindet, und
(b) wenn $R^{12}$ Methyl bedeutet und $R^{11}$ sich in 2-Stellung befindet, $R^{11}$ keine unsubstituierte exo-1-Azabicyclo[2,2,1]hept-3-yl-,exo-1-Azabicyclo[3,2,1]oct-3-yl- oder exo-1-Azabicyclo[3,2,1]oct-6-yl-Gruppe ist;
(c) wenn $R^1$ gegebenenfalls an einem Ringkohlenstoffatom oder Stickstoffatom durch $C_1$-$C_4$-Alkyl substituiertes 1,2,5,6-Tetrahydropyrid-3-yl oder Piperid-3-yl bedeutet, dann $R^1$ kein Wasserstoff, $-CF_3$, $-OR^7$ oder $C_1$-$C_6$-Alkyl, gegebenenfalls substituiert durch Halogen, -OH oder Phenyl, das wiederum gegebenenfalls durch Halogen, $C_1$-$C_6$-Alkoxy, $C_1$-$C_6$-Alkyl oder $-CF_3$ substituiert sein kann, bedeutet, umfassend
(A) das Umsetzen einer Verbindung der Formel $R^aCO.CH_2Y$, wobei Y Brom, Chlor oder Jod bedeutet, mit einer Verbindung der Formel $R^bCX.NH_2$, wobei X wie oben definiert ist, und einer der Reste $R^a$ und $R^b$ eine Gruppe der Formel $R^{11}$, wie oben definiert, bedeutet und der andere eine Gruppe der Formel $R^{12}$, wie oben definiert bedeutet, oder
(B) Dehydroxylieren einer Verbindung der Formel V:

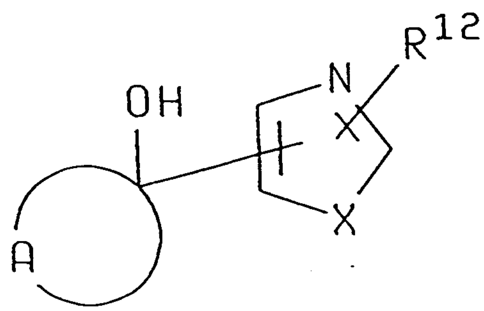
(V)

in der $R^{12}$ und X wie in oben definiert sind A den Rest eines azacyclischen oder azabicyclischen Ringsystems bedeutet, wobei das Ringsystem kondensiert, spiro oder überbrückt sein kann, enthaltend ein Stickstoffatom als einziges Heteroatom und 4 bis 10 Ringatome; oder
(C) zur Herstellung einer Verbindung der Formel II, in der X Schwefel bedeutet:
Cyclisieren eines Acylaminoketons der Formel $R^aCOCH_2NHCOR^b$, wobei $R^a$ und $R^b$ wie oben definiert sind, in Gegenwart eines dehydratisierenden Sulfids oder
(D) zur Herstellung einer Verbindung der Formel II, in der X Sauerstoff bedeutet:
Cyclisieren eines Acylaminoketons der Formel $R^aCOCH_2NHCOR^b$, wobei $R^a$ und $R^b$ wie oben definiert sind, in Gegenwart eines Dehydratisierungsmittels oder
(E) zur Herstellung einer Verbindung der Formel II, in der X Sauerstoff bedeutet:
Cyclisieren eines Ketals der Formel $R^aCOCH_2NHCR^bR^fR^g$, in der $R^a$ und $R^b$ wie oben definiert sind und $R^f$ und $R^g$ unabhängig voneinander $C_1$-$C_4$-Alkoxy bedeuten, oder
(F) zur Herstellung einer Verbindung der Formel II, in der X Sauerstoff bedeutet:
Umsetzen eines Aldehyds der Formel $R^aCHO$ mit dem Anion eines Isonitrils der Formel $L$-$CH_2N=C$-$(Z)R^b$, in der L eine austretende Gruppe bedeutet, Z Methoxy oder Methylthio bedeutet und $R^a$ und $R^b$ wie oben definiert sind; oder
(G) zur Herstellung einer Verbindung der Formel II, in der X Sauerstoff bedeutet und $R^{12}$ eine gegebenenfalls einfach substituierte Methylgruppe in 5-Stellung ist:
Cyclisieren eines Propargylamids der Formel XI:

$R^{11}CONHCH_2C{\equiv}CR^x$ (XI)

wobei $R^{11}$ wie oben definiert ist und $R^x$ eine solche Gruppe bedeutet, daß $-CH_2R^x$ die Gruppe $R^{12}$, wie oben definiert, bedeutet, durch Behandlung mit Quecksilberacetat in Essigsäure oder
(H) zur Herstellung einer Verbindung der Formel II, in der X Sauerstoff bedeutet und $R^{12}$ eine

gegebenenfalls einfach substituierte Methylgruppe in 4-Stellung bedeutet:
Umsetzung einer Cyanoverbindung der Formel $R^{11}CN$ mit einem Propargylalkohol der Formel $HOCH_2C{\equiv}CR^x$, wobei $R^{11}$ und $R^x$ wie oben definiert sind.

4. Verfahren nach Anspruch 3 zur Herstellung einer Verbindung der Formel III

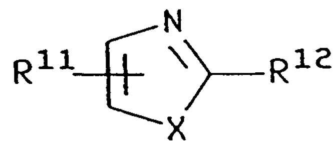

(III)

in der X, $R^{11}$ und $R^{12}$ wie in Anspruch 3 definiert sind.

5. Verfehren nach Anspruch 3 zur Herstellung einer Verbindung der Formel IV:

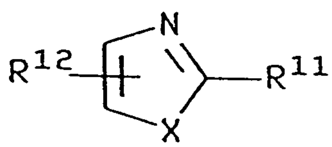

(IV)

wobei X, $R^{11}$ und $R^{12}$ wie in Anspruch 3 definiert sind.

6. Verfahren nach einem der Ansprüche 3 bis 5 zur Herstellung einer Verbindung, in der $R^{11}$ Tetrahydropyridin, 1-Azanorbornan, Chinuclidin oder 1-Azabicyclo[3,2,1]octan bedeutet, wobei jede der Gruppen $R^{11}$ durch $C_1$-$C_3$-Alkyl oder Hydroxy substituiert sein kann.

7. Verfahren nach einem der Ansprüche 3 bis 6 zur Herstellung einer Verbindung, bei der $R^{12}$ Wasserstoff, Methyl, Ethyl, Amino oder Hydroxy ist.

8. Verfahren nach einem der Ansprüche 3 bis 7 zur Herstellung einer Verbindung, ausgewählt aus
3-[4-(2-Amino-1,3-thiazol)-yl]chinuclidin;
3-[4-(1,3-Thiazol)-yl]chinuclidin;
3-[4-(2-Amino-1,3-thiazol)-yl]pyrrolidin;
3-[4-(2-Amino-1,3-thiazol)-yl]-1-methylpyrrolidon;
3-[4-(2-Methyl-1,3-thiazol)-yl]chinuclidin;
3-[4-(2-Hydroxy-1,3-thiazol)-yl]chinuclidin;
3-[5-(2-Methyl-1,3-thiazol)-yl]chinuclidin;
3-[4-(2-Amino-1,3-thiazol)-yl]-1-azabicyclo[2,2,1]heptan;
3-[5-(2-Methyl-1,3-oxazol)-yl]chinuclidin;
3-[5-(2-Methyl-1,3-oxazol)-yl]-1-azabicyclo[2,2,1]heptan;
3-[4-(2-Methyl-1,3-oxazol)-yl]chinuclidin;
3-[4-(2-Amino-1,3-oxazol)-yl]chinuclidin;
3-[2-(4-Methyl-1,3-thiazol)-yl]chinuclidin;
3-[2-(4-Methyl-1,3-oxazol)-yl]chinuclidin;
3-[2-(5-Methyl-1,3-oxazol)-yl]chinuclidin;
3-[2-(4-Methyl-1,3-thiazol)-yl]-1-azabicyclo[2,2,2]oct-2-en;
3-Hydroxy-3-[2-(4-methyl-1,3-oxazol)-yl]chinuclidin;
(+)-3-[2-(4-Methyl-1,3-oxazol)-yl]chinuclidin;
(-)-3-[2-(4-Methyl-1,3-oxazol)-yl]chinuclidin;
3-Hydroxy-3-[2-(1,3-oxazol)-yl]chinuclidin;
3-Hydroxy-3-[2-(4-ethyl-1,3-oxazol)-yl]chinuclidin;

3-[2-(4-Ethyl-1,3-oxazol)-yl]chinuclidin;
3-Hydroxy-3-[2-(4-methyl-1,3-thiazol)-yl]chinuclidin;
1-Methyl-3-[2-(5-methyl-1,3-oxazol)-yl]piperidin;
1-Methyl-3-[2-(5-methyl-1,3-oxazol)-yl]-1,2,5,6-tetrahydropyridin;
3-[2-(5-Methyl-1,3-oxazol)-yl]-1,2,5,6-tetrahydropyridin;
1-Methyl-3-[4-(2-methyl-1,3-thiazol)-yl]-1,2,5,6-tetrahydropyridin;
3-[4-(2-Methyl-1,3-thiazol)-yl]-1,2,5,6-tetrahydropyridin;
1-Methyl-3-[4-(2-methyl-1,3-oxazol)-yl]-1,2,5,6-tetrahydropyridin;
3-[4-(2-Methyl-1,3-oxazol)-yl]-1,2,5,6-tetrahydropyridin;
1-Methyl-3-[2-(4-methyl-1,3-oxazol)-yl]-1,2,5,6-tetrahydropyridin;
1-Methyl-3-[5-(2-methyl-1,3-oxazol)-yl]-1,2,5,6-tetrahydropyridin;
3-[2-(4-Methyl-1,3-oxazol)-yl]-1,2,5,6-tetrahydropyridin;
3-[5-(2-Methyl-1,3-oxazol)-yl]-1,2,5,6-tetrahydropyridin

und Salzen und Pro-Pharmaka davon.

**9.** Verfahren zur Herstellung eines pharmazeutischen Mittels, umfassend das Vermischen einer Verbindung, hergestellt nach einem der Ansprüche 3 bis 8 mit einem pharmazeutisch annehmbaren Träger.

**10.** Verfahren nach Anspruch 9, umfassend ferner den Einbau eines peripheren cholinergen Antagonisten in das Mittel.

**Revendications**

**Revendications pour les Etats contractants suivants : AT, BE, CH, DE, FR, GB, IT, LI, LU, NL, SE**

**1.** Utilisation de 1,3-oxazole ou 1,3-thiazole de formule structurale I ou un de leurs sels pharmaceutiquement acceptables ou un de leur précurseur :

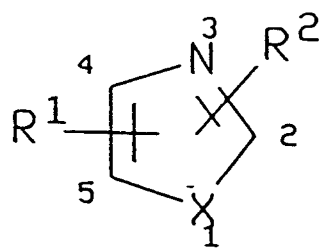

**(I)**

dans laquelle
X représente l'oxygène ou le soufre;
$R^1$ représente un cycle azacyclique ou azabicyclique non-aromatique, ce système peut être fusionné, spiro ou ponté, contenir un atome d'azote comme seul hétéroatome et de 4 à 10 atomes dans le cycle; et
$R^2$ représente un hydrogène, halogène, $-CF_3$, $-OR^7$, $-NR^7R^8$, $-NHOR^7$, $-NHNH_2$, -CN, $-CO_2R^7$ ou $-CONR^7R^8$ ou un groupe hydrocarbure substitué ou non substitué, saturé ou non saturé choisi parmi les alkyle $C_{1-15}$, alcényle $C_{2-15}$, alcynyle $C_{2-15}$, aryle et aralkyle; dans laquelle $R^7$ et $R^8$ représentent indépendamment l'hydrogène ou un alkyle $C_{1-2}$;
à la condition que :
(a) quand $R^2$ est l'hydrogène et $R^1$ est en position 2, alors $R^1$ est en configuration endo; et
(b) quand $R^2$ est le méthyle et $R^1$ est en position 2, alors $R^1$ ne représente pas un groupe exo-1-azabicyclo[2.2.1]-hept-3-yl,exo-1-azabicyclo[3.2.1] oct-3-yl-ou exo-1-azabicyclo[3.2.1]oct-6-yl;
(c) quand $R^1$ est le 1,2,5,6-tétrahydropyrid-3-yl ou pipérid-3-yl, éventuellement substitué sur un carbone du cycle ou un atome d'azote du cycle par un alkyle $C_{1-4}$, alors $R^2$ est différent de l'hydrogène, $-CF_3$, $-OR^7$ ou alkyle $C_{1-6}$ éventuellement substitué par un halogène, -OH ou phényle qui peut lui-même être substitué par un halo, alcoxy $C_{1-6}$, alkyle $C_{1-6}$ ou $CF_3$; pour préparer un

médicament utile au traitement de maladies mentales et neurologiques dont les manifestations cliniques sont dues à l'implication de neurones cholinergiques.

**2.** Utilisation d'un composé de formule I tel que défini à la revendication 1, ou un de ses sels pharmaceutiquement acceptables ou un de ses précurseurs pour préparer un médicament utile au traitement de conditions pénibles graves.

**3.** Composé de formule II, ou un de ses sels, ou un de ses précurseurs

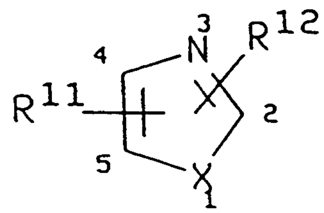

(II)

dans laquelle

X représente l'oxygène ou le soufre;

$R^{11}$ représente un cycle azacyclique ou azabicyclique non aromatique, ce système peut être fusionné, spiro ou ponté, contenir un atome d'azote comme seul hétéroatome et de 4 à 10 atomes dans le cycle; et

$R^{12}$ représente un hydrogène, halogène, $-CF_3$, $-OR^7$, $-NR^7R^8$, $-NHOR^7$, $-NHNH_2$, $-CN-CO_2R^7$ ou $-CONR^7R^8$ ou un groupe hydrocarbure éventuellement substitué, saturé ou non saturé choisi parmi les alkyle $C_{1-15}$, alcényle $C_{2-15}$, alcynyle $C_{2-15}$, aryle et aralkyle; dans laquelle $R^7$ et $R^8$ représentent indépendamment l'hydrogène ou un alkyle $C_{1-2}$;

à la condition que :

(a) quand $R^{12}$ est l'hydrogène et $R^{11}$ est en position 2, alors $R^{11}$ est en configuration endo; et

(b) quand $R^{12}$ est le méthyle et $R^{11}$ est en position 2, alors $R^{11}$ ne représente pas un groupe non substitué exo-1-azabicyclo[2.2.1]-hept-3-yl, exo-1-azabicyclo [3.2.1]-oct-3-yl ou exo-1-azabicyclo[3.2.1]oct-6-yl;

(c) quand $R^1$ est le 1,2,5,6-tétrahydropyrid-3-yle ou pipérid-3-yl, éventuellement substitué sur un carbone du cycle ou un atome d'azote du cycle par un alkyle $C_{1-4}$, alors $R^2$ est différent de l'hydrogène, $-CF_3$, $-OR^7$ ou alkyle $C_{1-6}$ éventuellement substitué par un halogène, -OH ou phényle qui peut lui-même être substitué par un halo, alcoxy $C_{1-6}$, alkyle $C_{1-6}$ ou $CF_3$.

**4.** Composé selon la revendication 3 représenté par la formule III

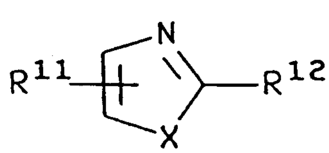

(III)

dans laquelle X, $R^{11}$ et $R^2$ sont tels que définis à la revendication 3.

**5.** Composé selon la revendication 3 représenté par la formule IV

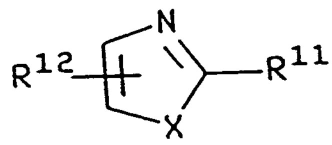

(IV)

dans laquelle X, $R^{11}$ et $R^{12}$ sont tels que définis à la revendication 3.

**6.** Composé selon l'une quelconque des revendications 3 à 5 dans lequel $R^{11}$ représente tétrahydropyridine, 1-azabornane, quinuclidine ou 1-azabicyclo[3.2.1]octane, l'un quelconque des groupes $R^{11}$ peut être substitué par alkyle $C_{1-3}$ ou hydroxy.

**7.** Composé sèlon l'une quelconque des revendications 3 à 6 dans lequel $R^{12}$ représente un hydrogène, méthyle, éthyle, amino ou hydroxy.

**8.** Composé choisi parmi :
3-[4-(2-amino-1,3-thiazol)-yl]pyrrolidine;
3-[4-(2-amino-1,3-thiazol)-yl]-1-méthylpyrrolidine;
3-[4-(2-méthyl-1,3-thiazol)-yl]quinuclidine;
3-[4-(2-hydroxy-1,3-thiazol)-yl]quinuclidine;
3-[5-(2-méthyl-1,3-thiazol)-yl]quinuclidine;
3-[4-(2-amino-1,3-thiazol)-yl]-1-azabicyclo[2.2.1]heptane;
3-[5-(2-méthyl-1,3-oxazol)-yl]quinuclidine;
3-[5-(2-méthyl-1,3-oxazol)-yl]-1-azabicyclo[2.2.1]heptane;
3-[4-(2-méthyl-1,3-oxazol)-yl]quinuclidine;
3-[4-(2-amino-1,3-oxazol)-yl]quinuclidine;
3-[2-(4-méthyl-1,3-thiazol)-yl]quinuclidine;
3-[2-(4-méthyl-1,3-oxazol)-yl]quinuclidine;
3-[2-(5-méthyl-1,3-oxazol)-yl]quinuclidine;
3-[2-(4-méthyl-1,3-thiazol)-yl]-1-azabicyclo[2.2.2]oct-2-ène;
3-hydroxy-3-[2-(4-méthyl-1,3-oxazol)-yl]quinuclidine;
(+)-3-[2-(4-méthyl-1,3-oxazol)-yl]quinuclidine;
(-)-3-[2-(4-méthyl-1,3-oxazol)-yl]quinuclidine;
3-hydroxy-3-[2-(1,3-oxazol)-yl]quinuclidine;
3-hydroxy-3-[2-(4-éthyl-1,3-oxazol)-yl]quinuclidine;
3-[2-(4-éthyl-1,3-oxazol)-yl]quinuclidine;
3-hydroxy-3-[2-(4-méthyl-1,3-thiazol)-yl]quinuclidine;
1-méthyl-3-[2-(5-méthyl-1,3-oxazol)-yl]pipéridine;
1-méthyl-3-[2-(5-méthyl-1,3-oxazol)-yl]-1,2,5,6-tétrahydropyridine;
3-[2-(5-méthyl-1,3-oxazol)-yl]-1,2,5,6-tétrahydropyridine;
1-méthyl-3-[4-(2-méthyl-1,3-thiazol)-yl]-1,2,5,6-tétrahydropyridine;
3-[4-(2-méthyl-1,3-thiazol)-yl]-1,2,5,6-tétrahydropyridine;
1-méthyl-3-[4-(2-méthyl-1,3-oxazol)-yl]-1,2,5,6-tétrahydropyridine;
3-[4-(2-méthyl-1,3-oxazol)-yl]-1,2,5,6-tétrahydropyridine;
1-méthyl-3-[2-(4-méthyl-1,3-oxazol)-yl]-1,2,5,6-tétrahydropyridine;
1-méthyl-3-[5-(2-méthyl-1,3-oxazol)-yl]-1,2,5,6-tétrahydropyridine;
3-[2-(4-méthyl-1,3-oxazol)-yl]-1,2,5,6-tétrahydropyridine;
3-[5-(2-méthyl-1,3-oxazol)-yl]-1,2,5,6-tétrahydropyridine;
et leurs sels et précurseurs.

**9.** Composition pharmaceutique comprenant un composé selon l'une quelconque des revendications 3 à 8 en association avec un véhicule pharmaceutiquement acceptable.

**10.** Composition pharmaceutique selon la revendication 9 comprenant en plus un antagoniste cholinergique périphérique.

**11.** Procédé de préparation d'un composé selon l'une quelconque des revendications 3 à 8, ce procédé comprend :

A) de faire réagir un composé de formule $R^aCO.CH_2Y$, dans lequel Y représente bromo, chloro ou iodo; avec un composé de formule $R^bCXNH_2$, dans lequel X est défini comme ci-dessus, et l'un des groupes $R^a$ et $R^b$ représente un groupe $R^{11}$ et l'autre représente un groupe $R^{12}$ tel que défini ci-dessus; ou

B) de déshydroxyler un composé de formule V :

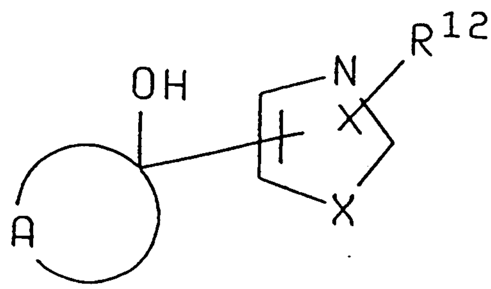

**(V)**

dans laquelle $R^{12}$ et X sont tels que définis ci-dessus, et A représente le reste d'un système de cycle azacyclique ou azabicyclique, ce système pouvant être fusionné, spiro ou ponté, qui contient un atome d'azote comme seul hétéroatome et de 4 à 10 atomes dans le cycle; ou

C) pour préparer un composé de formule II dans laquelle X représente le soufre :
cycliser une acylaminocétone de formule $R^aCOCH_2NHCOR^b$, dans laquelle $R^a$ et $R^b$ sont tels que définis ci-dessus, en présence d'un sulfure deshydratant; ou

D) pour préparer un composé de formule II dans laquelle X représente l'oxygène;
cycliser une acylaminocétone de formule $R^aCOCH_2NHCOR^b$, dans laquelle $R^a$ et $R^b$ sont tels que définis ci-dessus, en présence d'un agent deshydratant; ou

E) pour préparer un composé de formule II dans laquelle X représente l'oxygène;
cycliser un cétal de formule $R^aCOCH_2NHCR^bR^fR^g$ dans laquelle $R^a$ et $R^b$ sont tels que définis ci-dessus et $R^f$ et $R^g$ représentent indépendamnent un alcoxy $C_{1-4}$; ou

F) pour préparer un composé de formule II dans laquelle X représente l'oxygène;
faire réagir un aldéhyde de formule $R^aCHO$ avec l'anion d'un isonitrile de formule $L\text{-}CH_2N=C(Z)R^b$, dans laquelle L représente un groupe labile, Z représente le méthoxy ou méthylthio, et $R^a$ et $R^b$ sont tels que définis ci-dessus;

G) pour préparer un composé de formule II dans laquelle X représente un oxygène et $R^{12}$ représente un groupe méthyle éventuellement monosubstitué en position 5;
cycliser un amide propargylique de formule XI

$$R^{11}CONHCH_2C=CR^x \qquad (XI)$$

dans laquelle $R^{11}$ est tel que défini ci-dessus et $R^x$ est un groupe tel que $-CH_2R^x$ représente le groupe $R^{12}$ tel que défini ci-dessus, par traitement avec l'acétate mercurique dans l'acide acétique; ou

H) pour préparer un composé de formule II dans laquelle X représente l'oxygène et $R^{12}$ représente un groupe méthyle éventuellement mono-substitué en position 4 ; faire réagir un composé cyano de formule $R^{11}CN$ avec un alcool propargylique de formule $HOCH_2C{\equiv}CR^x$ dans laquelle $R^{11}$ et $R^x$ sont tels que définis ci-dessus.

**Revendications pour l'Etat contractant suivant : ES**

**1.** Utilisation de 1,3-oxazole ou 1,3-thiazole de formule structurale I ou un de leurs sels pharmaceutiquement acceptables ou un de leurs précurseurs :

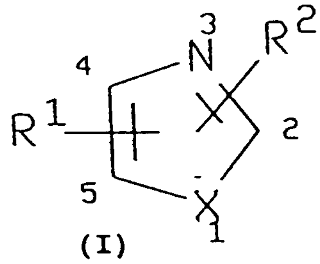

(I)

dans laquelle

- X représente l'oxygène ou le soufre;
- $R^1$ représente un cycle azacyclique ou azabicyclique non aromatique, ce système peut être fusionné, spiro ou ponté, contenir un atom d'azote comme seul hétéroatome et de 4 à 10 atomes dans le cycle; et
- $R^2$ représente un hydrogène, halogène, $-CF_3$, $-OR^7$, $-NR^7R^8$, $-NHOR^7$, $-NHNH_2$, -CN, $-CO_2R^7$ ou $-CONR^7R^8$ ou un groupe hydrocarbure substitué ou non substitué, saturé ou non saturé choisi parmi les alkyle $C_{1-15}$, alcényle $C_{2-15}$, alcynyle $C_{2-15}$, aryle et aralkyle; dans laquelle $R^7$ et $R^8$ représentent indépendamment l'hydrogène ou un alkyle $C_{1-2}$;

  à la condition que :

  (a) quand $R^2$ est l'hydrogène et $R^1$ est en position 2, alors $R^1$ est en configuration endo; et

  (b) quand $R^2$ est le méthyle et $R^1$ est en position 2, alors $R^1$ ne représente pas un groupe exo-1-azabicyclo [2.2.1]hept-3-yl, exo-1-azabicyclo-[3.2.1]-oct-3-yl ou exo-1-azabicyclo-[3.2.1]oct-6-yl;

  (c) quand $R^1$ est le 1,2,5,6-tétrahydropyrid-3-yl ou pipérid-3-yl , éventuellement substitué sur un carbone du cycle ou un atome d'azote du cycle par un alkyle $C_{1-4}$, alors $R^2$ est différent de l'hydrogène, $-CF_3$, $-OR^7$ ou alkyle $C_{1-6}$ éventuellement substitué par un halogène, -OH ou phényle qui peut lui-même être substitué par un halo, alcoxy $C_{1-6}$, alkyle $C_{1-6}$ ou $CF_3$; pour préparer un médicament utile au traitement de maladies mentales et neurologiques dont les manifestations cliniques sont dues à l'implication de neurones cholinergiques.

2. Utilisation d'un composé de formule I tel que défini à la revendication 1, ou un de ses sels pharmaceutiquement acceptables ou un de ses précurseurs pour préparer un médicament utile au traitement de conditions pénibles graves.

3. Procédé de préparation d'un composé de formule II ou un de ses sels ou précurseur :

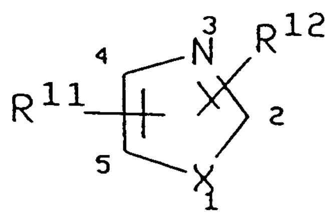

(II)

dans laquelle

- X représente l'oxygène ou le soufre;
- $R^{11}$ représente un cycle azacyclique ou azabicyclique non aromatique, ce système peut être fusionné, spiro ou ponté, contenir un atome d'azote comme seul hétéroatome et de 4 à 10 atomes dans le cycle; et
- $R^{12}$ représente un hydrogène, halogène, $-CF_3$, $-OR^7$, $-NR^7R^8$, $-NHOR^7$, $-NHNH_2$, -CN, $-CO_2R^7$ ou $-CONR^7R^8$ ou un groupe hydrocarbure éventuellement substitué, saturé ou non saturé choisi parmi les alkyles $C_{1-15}$, alcényle $C_{2-15}$, alcynyle $C_{2-15}$, aryle et aralkyle; dans

laquelle $R^7$ et $R^8$ représentent indépendamment l'hydrogène ou un alkyle $C_{1-2}$;

à la condition que :

(a) quand $R^{12}$ est l'hydrogène et $R^{11}$ est en position 2, alors $R^{11}$ est en configuration endo; et

(b) quand $R^{12}$ est le méthyle et $R^{11}$ est en position 2, alors $R^{11}$ ne représente pas un groupe non substitué exo-1-azabicyclo[2.2.1]hept-3-yl, exo-1-azabicyclo[3.2.1]oct-3-yl ou exo-1-azabicyclo[3.2.1]oct-6-yl;

(c) quand $R^1$ est le 1,2,5,6-tétrahydro-pyrid-3-yl ou pipérid-3-yl, éventuellement substitué sur un carbone du cycle ou un atome d'azote du cycle par un alkyle $C_{1-4}$, alors $R^2$ est différent de l'hydrogène, $-CF_3$, $-OR^7$ ou alkyle $C_{1-6}$ éventuellement substitué par un halogène, -OH ou phényle qui peut lui-même être substitué par un halo, alcoxy $C_{1-6}$, alkyle $C_{1-6}$ ou $CF_3$,

ce procédé comprend :

A) de faire réagir un composé de formule $R^aCO.CH_2Y$, dans lequel Y représente bromo, chloro ou iodo; avec un composé de formule $R^bCXNH_2$, dans lequel X est défini comme ci-dessus, et l'un des groupes $R^a$ et $R^b$ représente un groupe $R^{11}$ et l'autre représente un groupe $R^{12}$, tel que défini ci-dessus; ou

B) de déshydroxyler un composé de formule V

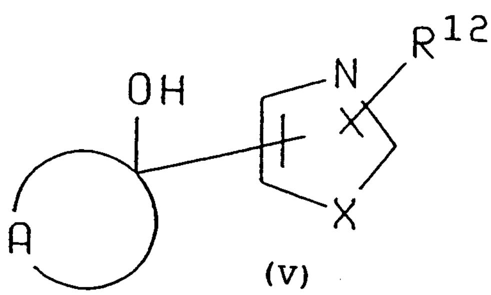

(V)

dans laquelle $R^{12}$ et X sont tels que définis ci-dessus, et A représente le reste d'un système de cycle azacyclique ou azabicyclique, ce système pouvant être fusionné, spiro ou ponté, qui contient un atome d'azote comme seul hétéroatome et de 4 à 10 atomes dans le cycle; ou

C) pour préparer un composé de formule II dans laquelle X représente le soufre,

cycliser une acylaminocétone de formule $R^aCOCH_2NHCOR^b$, dans laquelle $R^a$ et $R^b$ sont tels que définis ci-dessus, en présence d'un sulfure deshydratant; ou

D) pour préparer un composé de formule II dans laquelle X représente l'oxygène;

cycliser une acylaminocétone de formule $R^aCOCH_2NHCOR^b$, dans laquelle $R^a$ et $R^b$ sont tels que définis ci-dessus, en présence d'un agent deshydratant; ou

E) pour préparer un composé de formule II dans laquelle X représente l'oxygène;

cycliser un cétal de formule $R^aCOCH_2NHCR^bR^fR^g$ dans laquelle $R^a$ et $R^b$ sont tels que définis ci-dessus et $R^f$ et $R^g$ représentent indépendamment un alcoxy $C_{1-4}$; ou

F) pour préparer un composé de formule II dans laquelle X représente l'oxygène;

faire réagir un aldéhyde de formule $R^aCHO$ avec l'anion d'un isonitrile de formule $L-CH_2N=C-(Z)R^b$, dans laquelle L représente un groupe labile, Z représente le méthoxy ou méthylthio, et $R^a$ et $R^b$ sont tels que définis ci-dessus;

G) pour préparer un composé de formule II dans laquelle X représente un oxygène et $R^{12}$ représente un groupe méthyle éventuellement monosubstitué en position 5;

cycliser un amide propargylique de formule XI

$R^{11}CONHCH_2C=CR^x$ (XI)

dans laquelle $R^{11}$ est tel que défini ci-dessus et $R^x$ est un groupe tel que $-CH_2R^x$ représente le groupe $R^{12}$ tel que défini ci-dessus, par traitement avec l'acétate mercurique dans l'acide acétique; ou

H) pour préparer un composé de formule II dans laquelle X représente l'oxygène et $R^{12}$ représente

un groupe méthyle éventuellement monosubstitué en position 4; faire réagir un composé cyano de formule $R^{11}CN$ avec un alcool propargylique de formule $HOCH_2C{\equiv}CR^x$ dans laquelle $R^{11}$ et $R^x$ sont tels que définis ci-dessus.

**4.** Procédé selon la revendication 3 pour préparer un composé représenté par la formule III

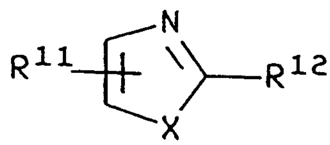

(III)

dans laquelle X, $R^{11}$ et $R^{12}$ sont tels que définis à la revendication 3.

**5.** Procédé selon la revendication 3 pour préparer un composé représenté par la formule IV

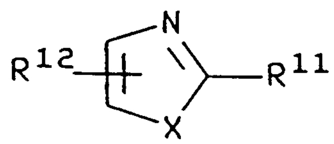

(IV)

dans laquelle X, $R^{11}$ et $R^{12}$ sont tels que définis à la revendication 3.

**6.** Procédé selon l'une quelconque des revendications 3 à 5 pour préparer un composé dans lequel $R^{11}$ représente tétrahydropyridine, 1-azabornane, quinuclidine ou 1-azabicyclo-[3.2.1]octane, l'un quelconque des groupes $R^{11}$ peut être substitué par alkyle $C_{1-3}$ ou hydroxy.

**7.** Procédé selon l'une quelconque des revendications 3 à 6 pour préparer un composé dans lequel $R^{12}$ représente un hydrogène, méthyle, éthyle, amino ou hydroxy.

**8.** Procédé selon l'une quelconque des revendications 3 à 7 pour préparer un composé choisi parmi :
3-[4-(2-amino-1,3-thiazol)-yl]pyrrolidine;
3-[4-(2-amino-1,3-thiazol)-yl]-1-méthylpyrrolidine;
3-[4-(2-méthyl-1,3-thiazol)-yl]quinuclidine,
3-[4-(2-hydroxy-1,3-thiazol)-yl]quinuclidine;
3-[5-(2-méthyl-1,3-thiazol)-yl]quinuclidine;
3-[4-(2-amino-1,3-thiazol)-yl]-1-azabicyclo[2.2.1]heptane;
3-[5-(2-méthyl-1,3-oxazol)-yl]quinuclidine;
3-[5-(2-méthyl-1,3-oxazol)-yl]-1-azabicyclo[2.2.1]heptane;
3-[4-(2-méthyl-1,3-oxazol)-yl]quinuclidine;
3-[4-(2-amino-1,3-oxazol)-yl]quinuclidine;
3-[2-(4-méthyl-1,3-thiazol)-yl]quinuclidine;
3-[2-(4-méthyl-1,3-oxazol)-yl]quinuclidine;
3-[2-(5-méthyl-1,3-oxazol)-yl]quinuclidine;
3-[2-(4-méthyl-1,3-thiazol)-yl]-1-azabicyclo[2.2.2]oct-2-ène;
3-hydroxy-3-[2-(4-méthyl-1,3-oxazol)-yl]quinuclidine;
(+)-3-[2-(4-méthyl-1,3-oxazol)-yl]quinuclidine;
(-)-3-[2-(4-méthyl-1,3-oxazol)-yl]quinuclidine;
3-hydroxy-3-[2-(1,3-oxazol)-yl]quinuclidine;
3-hydroxy-3-[2-(4-éthyl-1,3-oxazol)-yl]quinuclidine;
3-[2-(4-éthyl-1,3-oxazol)-yl]quinuclidine;

3-hydroxy-3-[2-(4-méthyl-1,3-thiazol)-yl]quinuclidine;
1-méthyl-3-[2-(5-méthyl-1,3-oxazol)-yl]pipéridine;
1-méthyl-3-[2-(5-méthyl-1,3-oxazol)-yl]-1,2,5,6-tétrahydropyridine;
3-[2-(5-méthyl-1,3-oxazol)-yl]-1,2,5,6-tétrahydropyridine;
1-méthyl-3-[4-(2-méthyl-1,3-thiazol)-yl]-1,2,5,6-tétrahydropyridine;
3-[4-(2-méthyl-1,3-thiazol)-yl]-1,2,5,6-tétrahydropyridine;
1-méthyl-3-[4-(2-méthyl-1,3-oxazol)-yl]-1,2,5,6-tétrahydropyridine;
3-[4-(2-méthyl-1,3-oxazol)-yl]-1,2,5,6-tétrahydropyridine;
1-méthyl-3-[2-(4-méthyl-1,3-oxazol)-yl]-1,2,5,6-tétrahydropyridine;
1-méthyl-3-[5-(2-méthyl-1,3-oxazol)-yl]-1,2,5,6-tétrahydropyridine;
3-[2-(4-méthyl-1,3-oxazol)-yl]-1,2,5,6-tétrahydropyridine;
3-[5-(2-méthyl-1,3-oxazol)-yl]-1,2,5,6-tétrahydropyridine.

**9.** Procédé pour préparer une composition pharmaceutique qui comprend de mélanger un composé préparé selon l'une quelconque des revendications 3 à 8 avec un véhicule pharmaceutiquement acceptable.

**10.** Procédé selon la revendication 9 qui comprend en outre d'incorporer un antagoniste cholinergique périphérique dans la composition.